# FASCICULE DE BREVET EUROPEEN

(11) **EP 0 511 036 B1**
(45) Date de publication et mention de la délivrance du brevet: **15.10.1997**
(21) Numéro de dépôt: 92400866.7
(22) Date de dépôt: 27.03.1992
(51) Int. Cl.: C07C 35/08, C07C 39/27, C07D 303/08, C07D 303/34

(54) **Intermédiaires de synthèse présentant un cycle hexanique et leurs procédés de préparation**
Synthesezwischenprodukte mit einem Hexanring und Verfahren zu ihrer Herstellung
Synthesis intermediaries with an hexanic ring, and process for their preparation

(30) Priorité: 17.04.1991 FR 9104700; 17.04.1991 FR 9104701
(43) Date de publication de la demande: 28.10.1992
(73) Titulaire: RHONE-POULENC CHIMIE, 92408 Courbevoie Cédex (FR)
(72) Inventeur: Duhamel, Lucette, F-76130 Mont-Saint-Aignan (FR); Leblond, Bertrand, F-76000 Rouen (FR); Duhamel, Pierre, F-76130 Mont-Saint-Aignan (FR); Poirier, Jean-Marie, Saint Martin du Vivier, F-76160 Darnetal (FR)
(74) Mandataire: Ricalens, François

(56) Documents cités:
- DE-B- 1 186 846
- DE-B- 1 221 622
- DE-B- 1 243 184
- GB-A- 1 022 203
- CHEMICAL ABSTRACTS, vol. 102, no. 19, 13 Mai 1985, Columbus, Ohio, US; abstract no. 166348U, JAMSHAID MUHAMMAD ET AL.: 'Reduction of tetrachlorocyclohexanone with metal hydrides' page 582 ;colonne 1 ;

## Description

La présente invention a pour objet de nouveaux intermédiaires de synthèse permettant d'accéder à des produits réputés difficiles d'accès et notamment à des dérivés benzéniques halogénés.

La présente invention concerne plus particulièrement des composés de synthèse dérivé de la cyclohexanone et notamment par substitution des positions en a par des atomes d'halogènes ou de pseudo-halogènes.

A titre d'exemple et pour montrer l'importance de ces produits, ces dérivés halogénés permettent d'obtenir des dihalo-2,6 anilines qui sont des intermédiaires utilisables pour obtenir des dérivés phénylacétiques, lesquels peuvent avoir des propriétés antiphlogistiques, analgésiques et antipyrètiques. En ce qui concerne la transformation des tétrahomohalo-2,2,6,6 cyclohexanone, on peut se référer au Brevet Européen publié sous le n° 0 313740.

En ce qui concerne leur intervention dans la synthèse des médicaments mentionnés ci-dessus, on peut se référer à la Demande de Brevet Japonaise publiée après examen, N° 23 418/1967. Malgré l'intérêt des cyclohexanones, tétra(pseudo)halogénées, les procédés de synthèse permettant d'accéder à ces produits, soit sont compliqués, soit n'ont jamais été décrits.

Ainsi, la synthèse de la tétrafluoro-2,2,6,6 cyclohexanone n'a jamais été décrite, non plus que, à la connaissance de la demanderesse le produit lui-même.

C'est pourquoi un des buts de la présente invention est de fournir de nouveaux intermédiaires réactionnels permettant d'avoir accès à des dérivés aromatiques halogénés notamment les dihalogénés et les halogéno phénols.

Un autre but de la présente invention est de fournir des dérivés cyclohexaniques extrêmement polyvalents pour obtenir divers composés organiques.

Un autre but de la présente invention est de fournir des procédés de synthèse permettant l'obtention des composés intermédiaires spécifiés ci-dessus.

Un autre but de la présente invention est de fournir des procédés permettant l'aromatisation des produits ci-dessus (nouveaux intermédiaires réactionnels).

Ces buts et d'autres sont atteints au moyen de dérivés époxydes cyclohexaniques. Ces composés selon l'invention répondent à la formule générale (II) suivante : 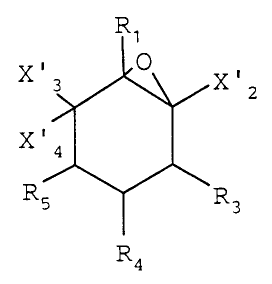
→ dans laquelle X'₂, X'₃ et X'₄ (qui correspondent un à un à X₂, X₃ et X₄ ou X₁, X₂ et X₃ de la formule I) semblables ou différents représentent un halogène ou un pseudo-halogène, de préférence un halogène avantageusement le chlore et le fluor avec la condition que lorsque R₁ est hydroxyle, cyano, amido, imido, éthoxyle, benzyloxyle, cyclohexyle et tertiobutyle, tous les halogènes ne peuvent être simultanément du chlore et R₄, R₃ et R₅ simultanément égal à H ;
→ dans laquelle R1 représente un hydrogène, une chaîne hydrocarbonée telle que par exemple une chaîne alcoyle, une chaîne aromatique ou un groupement alcoxyle, carboxyle ou acyloxyle, voire hydroxyle ;
→ dans laquelle R4 représente un hydrogène, un atome de fluor, une chaîne hydrocarbonée telle que par exemple une chaîne alcoyle, une chaîne aromatique ou un groupement carboxyle, carboxamide ou bien un radical relié au cycle hexanique par un chalcogène ou par élément de la colonne V de préférence de la première période, tel qu'un groupement amido, alcoxyle ou acyloxyle ;
→ dans laquelle les radicaux R3 et R5 différents ou de préférence semblables, représentent un atome de fluor, ou de préférence d'hydrogène, ou également des chaînes hydrocarbonées telles que définies ci-dessus pour R4.

Par pseudo halogènes on entend les groupements dont les anions font d'excellents groupes partants. plus précisement on vise les acides oxygénés dont la constante de Hammett démontre une acidité au moins égale à celle de de l'acide formique de préférence de l'acide trifluoroacétique comme paradigme des pseudo halogènes les sulfonyloxyles issus de l'acide triflique ou paratoluenesulfonique ainsi que les acide carboxyliques très acides tels que les acides alpha halogénés, de préférence alpha fluorés.

Le terme alcoyle doit ici être pris dans son acception étymologique Cf dictionnaire Larousse en trois volumes (1970) et non dans le sens du terme anglosaxon alkyle tel que défini très restrictivement par les règles de l'IUPAC. A titre paradigmatique on peut citer comme appartenant à cette famille les alkyles (selon la définition du dictionnaire de la Chimie de Clément Duval, publié aux Presses Scientifiques Internationales) les cycloalkyles,les aralkyles qui peuvent être substitués ou fonctionalisés dès lors que substituants ou fonctions sont sensiblement inertes aux réactifs selon la présente invention par exemple alcoyles derivés d'hémiéthers de polyéthylène glycols.

Bien qu'il n'y ait pas de limite stricte, il est rare que le nombre de carbones par substituants excède 15 et même 10. Le nombre total des carbones des substituants pris dans leur ensemble dépasse rarement 30 en général 20.

Dans la formule (II) ci-dessus les hydrogènes ne sont pas figurés et la numérotation des carbones du cycle correspond aux indices des radicaux R.

Une des propriétés les plus intéressantes des époxydes selon la présente invention est qu'ils s'ouvrent toujours vers le carbone central, c'est-à-dire que le carbone numéroté 1 dans la présente description et entouré de carbones substitués par des halogènes ou pseudo-halogènes porte la fonction alcool après ouverture de l'époxyde.

Les époxydes ci-dessus, dont les voies d'accès ne sont pas décrites dans l'état antérieur de la technique, même de manière très générale, peuvent être préparés par action d'une base sur un alcool de formule générale I ou I' .

L'époxyde se fait de préférence par attaque de l'anion correspondant à l'alcool, sur un carbone vicinal de celui qui porte l'alcool. Le choix du groupe partant quant à sa nature et à sa position est déterminé de la manière suivante :
- le groupe partant est en position "trans" par rapport à la fonction hydroxyle ;
- lorsque deux groupes partants sont possibles, c'est le meilleur groupe partant qui sera très préférentiellement éjecté ;
- lorsque les deux groupes partants possibles sont les mêmes, celui se trouvant sur le même carbone que l'halogène le plus électronégatif sera celui qui sera très préférentiellement éjecté.

Les époxydes peuvent être préparés à partir d'alcools portant sur le même carbone que la fonction alcool, aussi bien une chaîne (hydro)carbonée qu'un hydrogène.

La réaction d'épicyclisation, parfois designée ci-après sous le vocable d'époxydation, a avantageusement lieu dans les conditions suivantes:
- dans une phase polaire, de préférence aqueuse, hydroorganique si la solubilité du dérivé de formule I ou I' le rend souhaitable ; il est très préférable que ladite phase polaire soit susceptible de dissoudre, au moins partiellement, à la fois la base et l'alcool I ou I' ; avantageusement la solubilité du substrat alcoolique est au moins égale à 10⁻³ M plus préférentiellement à 10⁻² M et celle de la base à 10⁻³ N plus préférentiellement à 10⁻² N ;
- à une température comprise entre le point de fusion commençante du mélange réactionnel et 100°C de préférence entre 0°C et 50°C, en général entre 0°C et l'ambiante (environ 20°C) ;
- on utilise une base forte dont le pKa associé est au moins égal à 10 de préférence à 12, plus préférentiellement très forte (telle que un hydroxyde alcalin ou un hydroxyde de tétra alcoylammonium) en quantité au moins égale à la quantité stoechiométrique (à moins de ne vouloir réaliser la réaction que partiellement), de préférence en excès ;
- lorsque l'on désire une réaction rapide la concentration en base soluble excédentaire par rapport à la quantité stoechiométrique est avantageusement au moins décinormale, de préférence au moins normale ;
- la base est ajoutée progressivement, notamment pour faciliter la régulation de la température ;
- l'époxyde étant plus lipophile que l'halohydrine il est possible de réduire la possibilité d'éventuelle(s) réaction(s) parasite(s) en réalisant la réaction en milieu biphasique pendant tout ou partie de la réaction ; on utilise alors des solvants sensiblement inertes, bons solvants des époxydes cyclohexaniques et non miscibles en toutes proportions avec la phase aqueuse tel que par exemple les éthers, les esters, les organohalogénés ou leurs mélanges.

Les alcools cyclohexaniques précurseurs , directs ou indirects, des époxydes selon la présente invention présentent la formule (I'), où les hydrogènes ne sont pas figurés, ci-après : 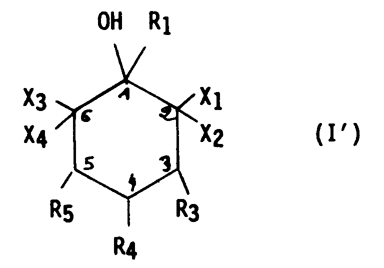
dans laquelle X₁, X₂, X₃ et X₄ semblables ou différents représentent un halogène ou un pseudo-halogène, avantageusement un halogène de préférence le chlore et le fluor ;
dans laquelle R₁ représente un hydrogène, une chaîne hydrocarbonée telle que par exemple une chaîne alcoyle, une chaîne aromatique ou un groupement alcoxyle, carbonyle, carboxyle ou acyloxyle, hydroxyle ; dans laquelle R₄ représente un hydrogène, un atome de fluor, une chaîne hydrocarbonée telle que par exemple une chaîne alcoyle, une chaîne aromatique ou un groupement alcoxyle ou acyloxyle :
dans laquelle les radicaux R₃ et R₅ différents ou de préférence semblables, représentent un atome de fluor, ou de préférence d'hydrogène, ou également des chaînes hydrocarbonées telles que définies ci-dessus pour R₄ ; ou
   présentent la formule (I) semblable à la formule (I') mais avec la condition supplementaire que lorsque R₁ est hydroxyle, tous les halogènes ne peuvent être simultanément du chlore ou du brome et lorsque R₁ est hydrogène, cyano, amido, tous les halogènes ne peuvent être simultanément du chlore.

Les produits équivalents au diols-1,1, tels la cétone-1 sont également visés par la présente invention. Les composés de formule I ou I' ci-dessus, différrents des diols-1,1 ou équivalents (cétones), peuvent selon le cas être obtenus respectivement par réduction des cétones correspondantes, en général sous forme hydratée, par action d'organometalliques, ou par ouverture des époxydes. Pour ce qui est des diols-1,1 ou équivalents par oxydation des alcools correspondants.

En général, l'alcool *initial* est un alcool obtenu par réduction d'un dérivé tétrahomohalogéné de la cyclohexanone, quand il existe. Celui dont l'accès est le mieux connu est celui obtenu par réduction de la tétrachloro-2,2,6,6-cyclohexanone. Cette réduction est connue de l'homme de métier et l'on peut notamment se référer aux articles suivants :
"Le travail" de TANAKA et coll. Y. KAWAZOE, T.TAGUCHI, YAKUGAKU ZASSHI (Japan) 1975, 95, 238, utilisant le mélange formiate alcalin/acide formique à 160-170°C. Le travail de M. JAMSHAID, M. Alam, J. Pharm. (Lahore) 1983 4(1-2), 101-3. C.A. 102, 166348u utilisant l'hydrure double de lithium et d'aluminium ou le borohydrure de sodium.

Toutefois, *au cours de la recherche qui à mené à* la présente invention, on a pu montrer que l'on pouvait *réduire les composés de formule I' avec R*_{*1*} *= OH , ou ses équivalents (cétone...) en utilisant*
- l'hydrure double du lithium et d'aluminium en présence d'éthers- *;*
- le chlorure d'isopropyl magnésium à -20° C *;*
- Mais une des meilleures manières de préparer par réduction ces tétrahalogénos alcools, est d'utiliser les borohydrures, avantageusement alcalins, en général de sodium pour des raisons d'économie, en présence d'un mélange entre un éther, avantageusement diéthylique et un alcool, de préférence méthylique à température ambiante, c'est-à-dire, à une température comprise entre 0 et 50°C, de préférence entre 15 et 30°C.

Le rapport préféré entre l'éther et l'alcool, exprimé en volume, est avantageusement compris entre 10 et 1, de préférence aux alentours de 10 (dans la présente description, comme d'ordinaire, les zéros de position ne sont pas réputés chiffres significatifs).

*Selon l'invention, c*es alcools peuvent aussi, et surtout, être obtenus par ouverture des époxydes correspondants (et objet de la présente invention), au moyen d'un ion halogénure ou pseudohalogénure rendu nucléophile.

La réaction d'ouverture de l'époxyde se fait selon des règles qui ont été déterminées au cours de l'étude qui a mené à la présente invention.

Ces règles peuvent être résumées par application des indications ci-après.

La réaction se fait comme si le mécanisme en cause n'était pas une substitution nucléophile d'ordre deux (SN2), c'est-à-dire sans inversion de configuration. En d'autres termes, après réaction l'halogène ou pseudo halogène se trouve en configuration cis par rapport à l'alcool issu du pont oxygène, ainsi tout se passe comme si la liaison entre le carbone en position vicinale du carbone 1 (c'est-à-dire l'un des carbones 2 ou 6) et l'oxygène ne bougait pas, mais que simplement au lieu de relier le carbone à l'oxygène de l'oxirane, elle reliait le carbone à un halogène ou à un pseudohalogène.

Il est ainsi possible de faire une halohydrine avec tout halogène ou pseudohalogène en utilisant les techniques selon la présente invention. Il existe toutefois des différences : Les halogènes considérés comme groupe partant peuvent être classés dans l'ordre des numéros atomiques croissants des halogènes : plus leur rang est élevé, meilleurs ils sont. A contrario plus ils sont légers, meilleure est leur nucléophilie.

Plus spécifiquement, selon la présente invention, ces époxydes sont ouverts par des acides de Brönstedt ou de Lewis comportant l'halogénure ou le pseudo-halogénure dans sa molécule.

Les acides de Lewis préférés sont soit ceux qui correspondent aux trihalogénures des éléments de la colonne III B de la classification périodique des éléments (définie par la Société Chimique de France, dans le supplément au Bull. Soc. Chim. Fr. N° 1 Janvier 1966), soit ceux susceptibles d'exister sous la forme de divers étherates (ces derniers peuvent être issus d'éthers symétriques ou non, cycliques ou non), soit ceux possédant les deux propriétés.

Les acides de Brönstedt préférés sont ceux dont l'ion halogénure ou pseudohalogénure constitue la base associée, avantageusement pour en faciliter la manipulation en présence d'une amine tertiaire avantageusement de faible basicité, de préférence les amines réputées tertiaires et qui contiennent comme source de base un atome d'azote inséré dans un cycle aromatique, comme par exemple les pyridines éventuellement substituées comme par exemple les picolines ou comme par exemple, les quinoléines.

L'utilisation d'un acide de Bronstedt étant plus simple à mettre en oeuvre, c'est cette dernière qui est en général préférée. Toutefois les acides de lewis donnent en principe des réactions plus sélectives.

Quoique les conditions opératoires dépendent des quantités et des spécificités des réactifs et des substrats, il est possible d'indiquer plus à titre de lignes directrices, qu'à titre de règles strictes les conditions opératoires, indépendamment les unes des autres, considérées comme préférées exposées ci-après.

La température est avantageusement comprise entre le point de fusion commençante et 100°C, de préférence entre 0°C et 50°C, le plus couramment au alentours de la température ambiante (environ 20°C). Lorsque l'on utilise un acide de lewis, notamment pour BF₃ les températures à prendre en compte sont plus élevées, la zône préférée et alors aux alentours de 100°C. Il est préféré de commencer la réaction à basse température, c'est-à-dire dans la zone basse des fourchettes spécifiées ci-dessus, puis laisser lentement la température s'élever jusqu'à la temperature désirée en général l'ambiante.

La pression est peu importante ; pour des raisons de simplicité, on utilise en général la pression atmosphérique. Toutefois si l'on désire réguler une température par un reflux, on peut aisément travailler sous pression réduite ou sous pression supérieure à la pression atmosphérique.

On utilise de préférence des solvants suffisamment polaires pour bien dissoudre les époxydes ainsi que les alcools obtenus selon cette technique. Parmi les solvants préférés, il convient de citer les solvants halogénés, en général chlorés, tels que le chlorure de méthylène, les dichloroéthanes notamment le dichloro-1,2 éthane, ou le trichloréthylène. Si l'on désire purifier le mélange réactionnel pour éliminer les sous-produits non désirés de la réaction, on peut utiliser des techniques classiques de séparation telles que par exemple la chromatographie éclair sur silice.

Pour ce qui concerne les paramètres non spécifiés, ils sont connus de l'homme de métier et on peut se référer aux exemples pour déterminer les domaines d'utilisation optimale des paramètres.

Cette sélectivité d'attaque, cette remarquable régiosélectivité, permet ainsi de changer progressivement tous les halogènes par une succession de formation et d'ouverture d'époxydes.

Afin de mieux expliciter les mécanismes, on trouve en pages 8a à 8e des schémas réactionnels montrant le rôle de la géométrie des molécules dans le remplacement progressif des halogènes ou pseudohalogènes.
Ces successions d'ouverture et de fermeture d'époxyde permettent de bien maîtriser de manière très sélective les subtitutions des halogènes entre eux. Ceci est parfaitement exemplifié par l'inversion de la configuration montrée dans les équations du schéma de la page 8 d. Ici le problème est de faire un composé di-fluoré comportant un fluor en position 2 et un fluor en position 6. Cette disposition permet de faciliter l'aromatisation pour donner un des difluorobenzènes éventuellement substitués avec de meilleurs rendements que le composé trifluoré.

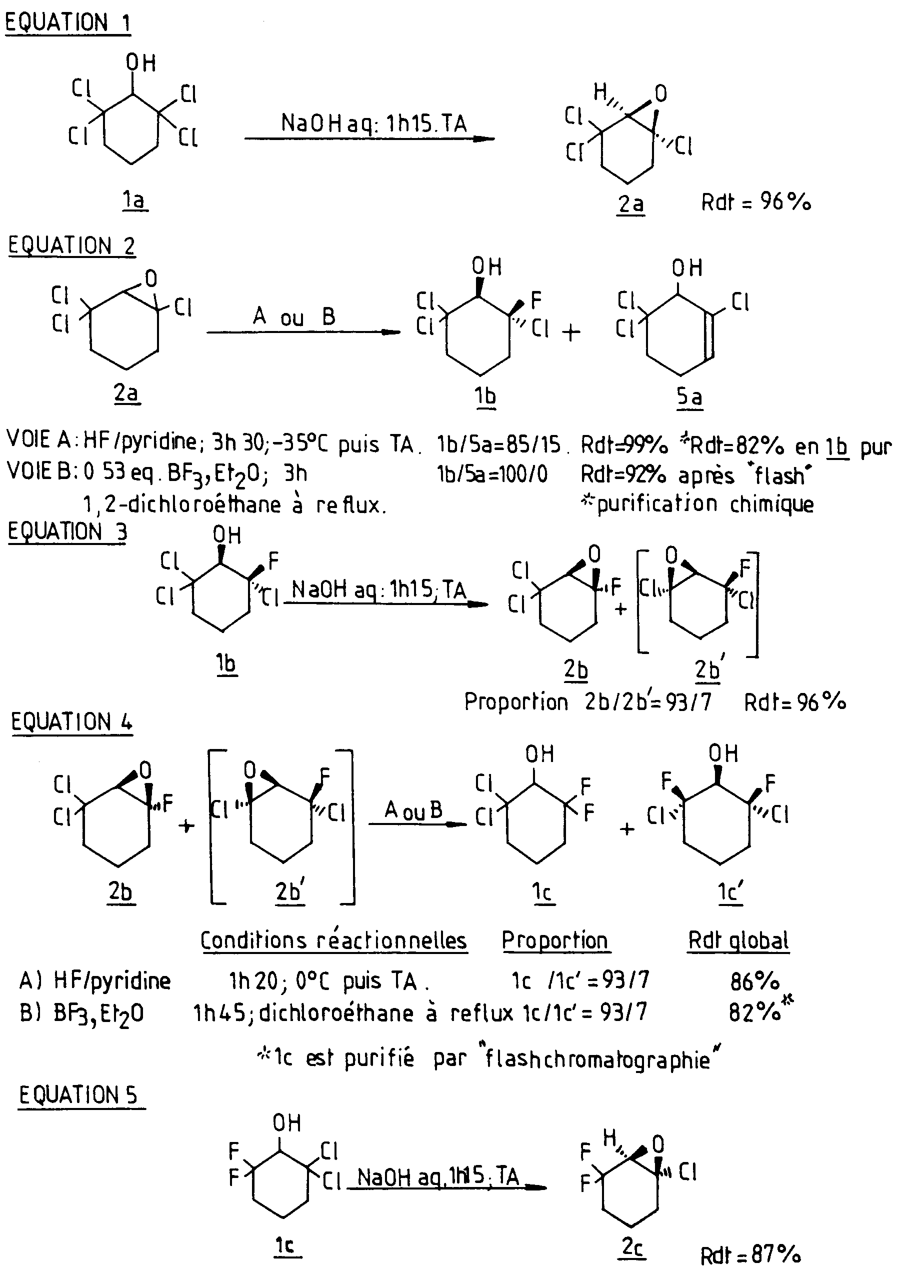

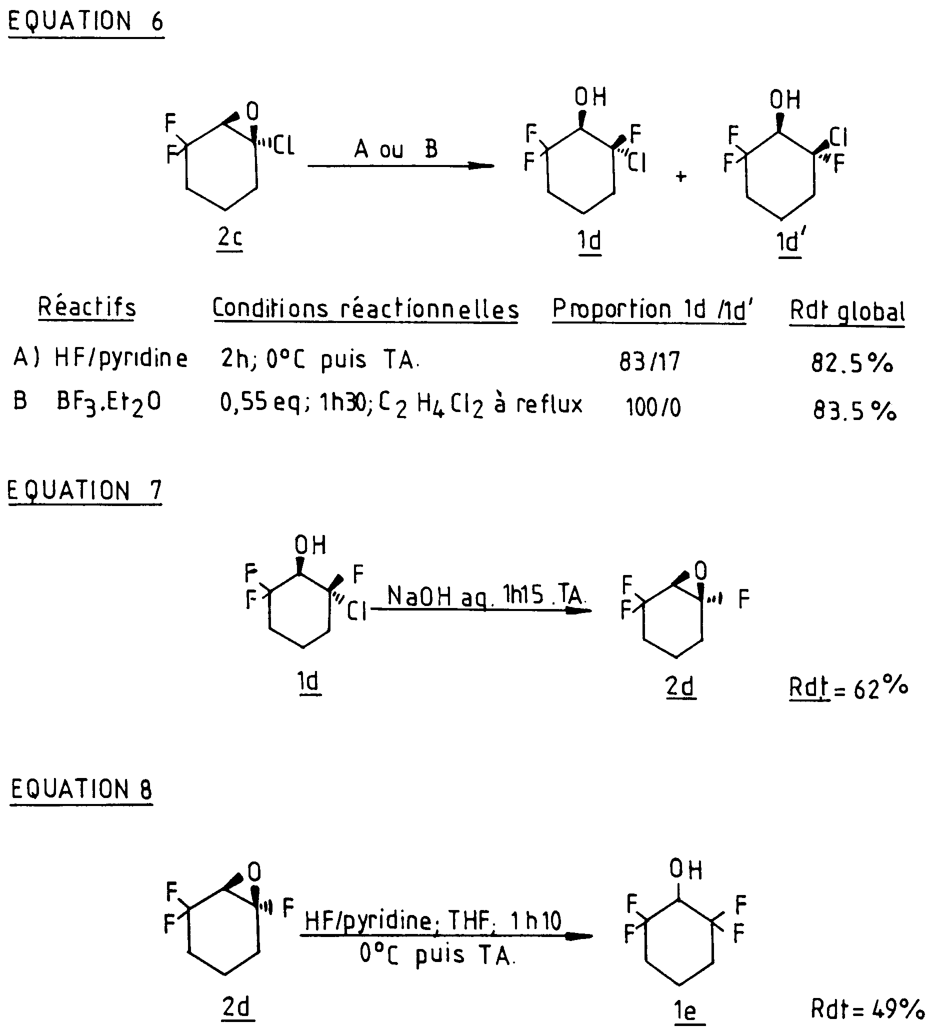

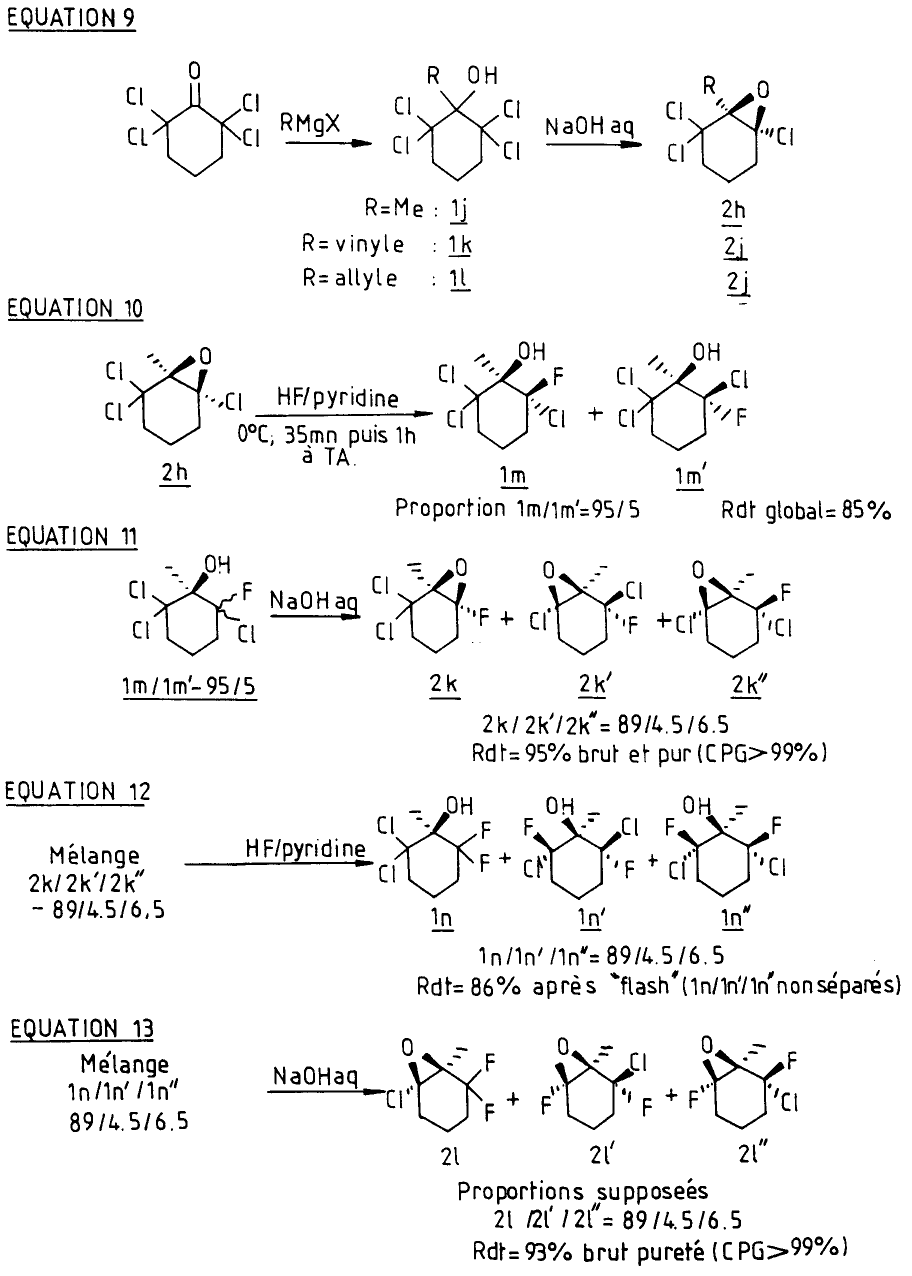

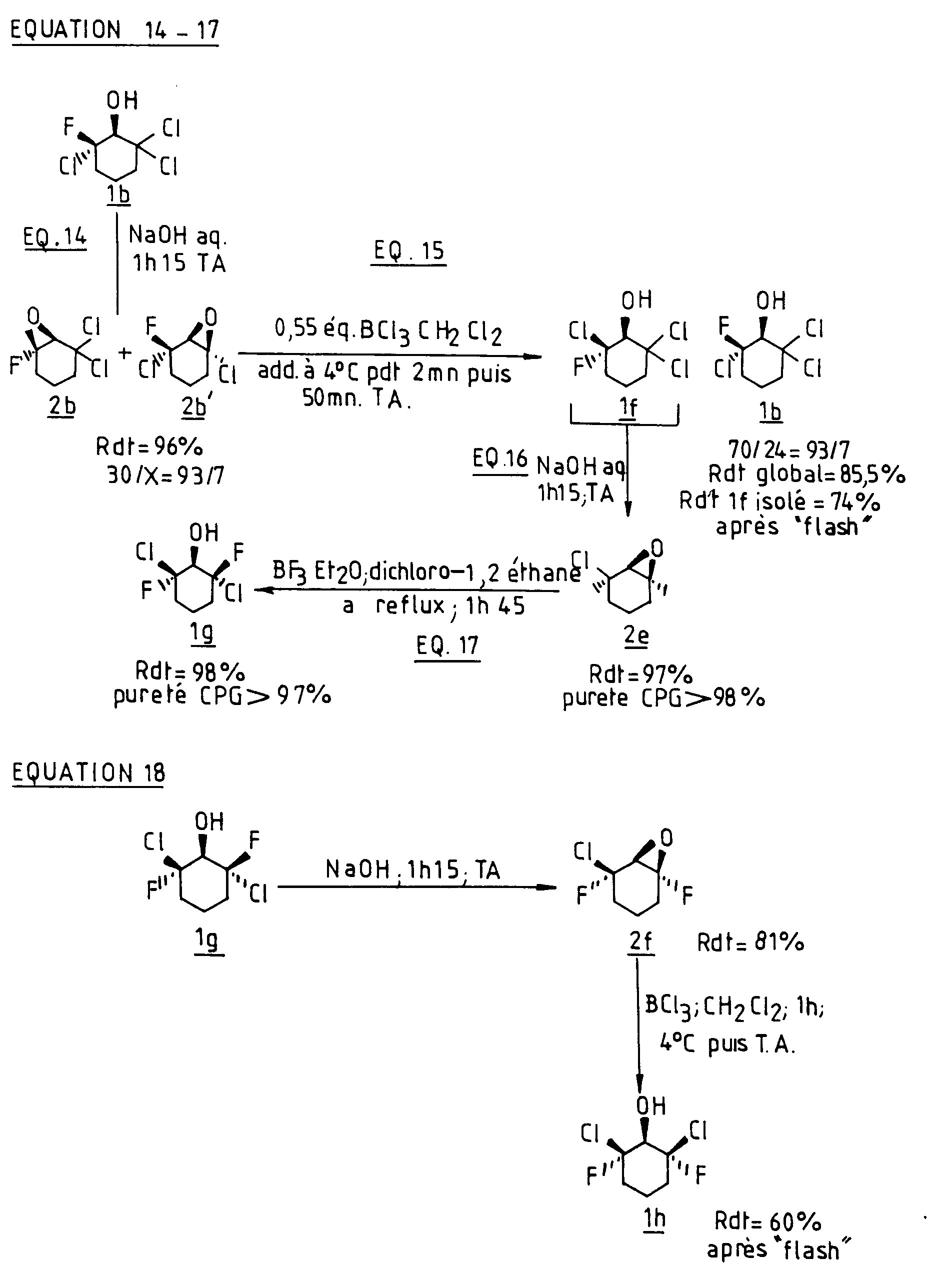

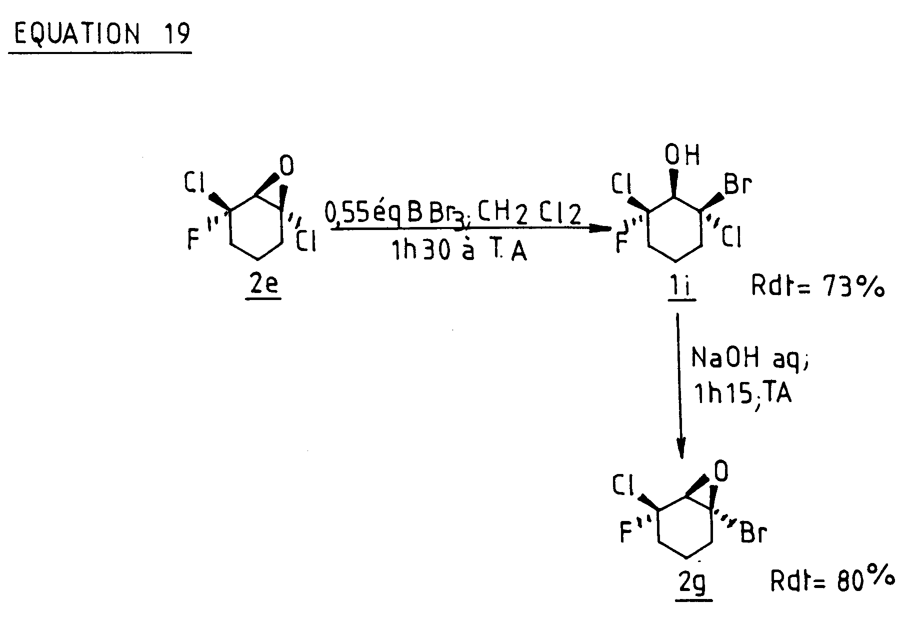

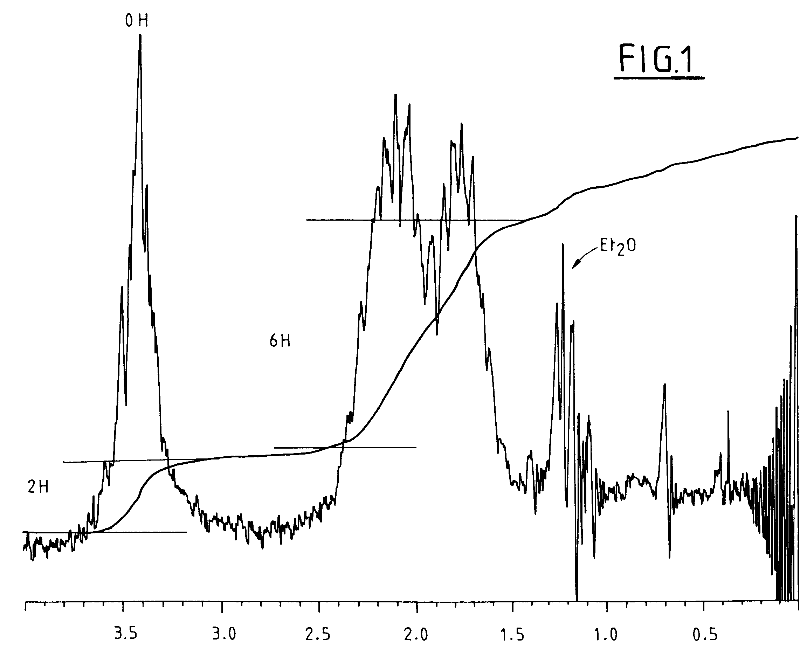

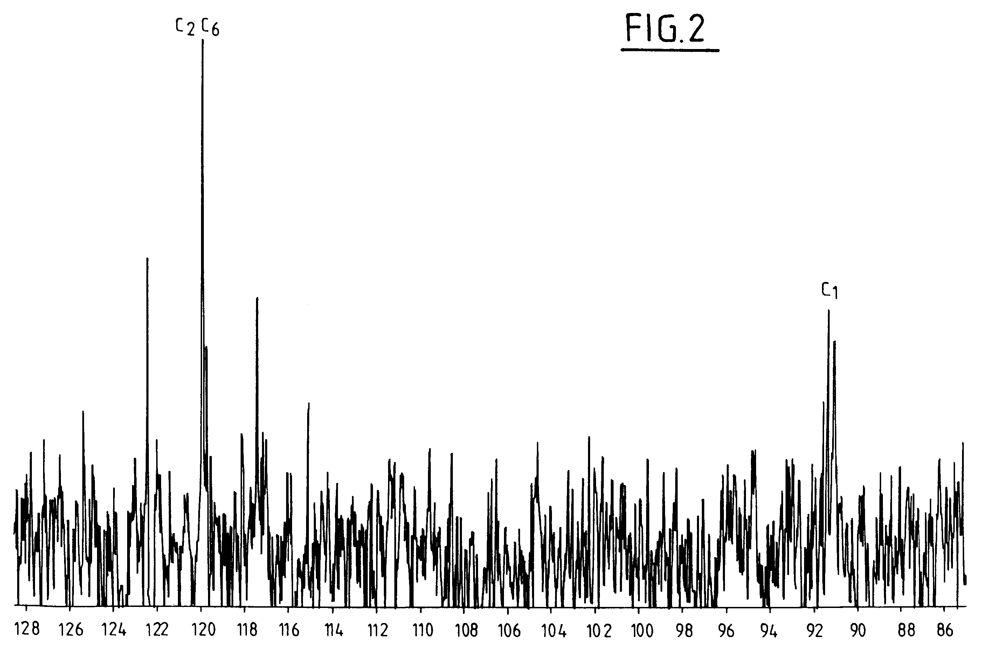

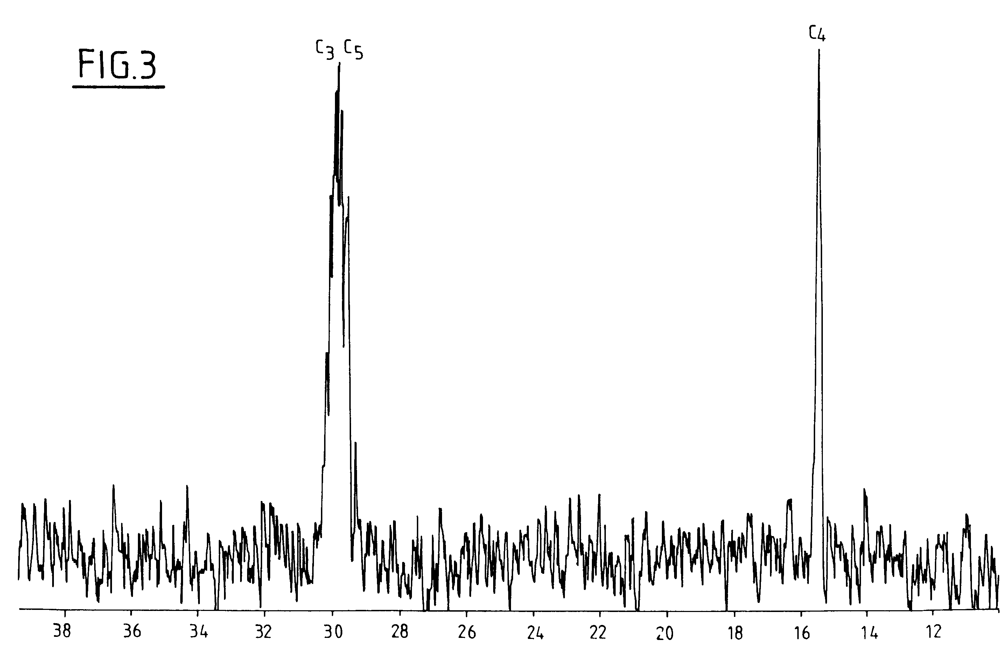

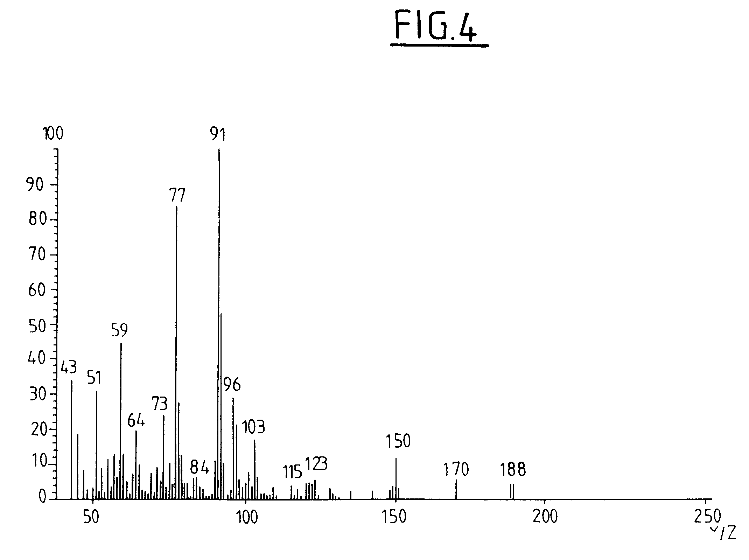

Les figures 1 à 4 représentent respectivement le spectre RMN du proton du carbone 13 et le spectre de masse du tétrafluoro 2,2,6,6 cyclohexane diol 1,1 .

### NUMEROTATION UTILISEE :

- Alcool 1a - 1n
- Epoxydes : 2a - 2l
- Hydrates : 3a - 3e
- Aromatiques : 4a - 4i
- Alcools allyliques : 5a - 5b
- Cétones trihalogénées : 6a - 6b

### COMMENTAIRES DES SCHEMAS DES PAGES 8a A 8e

### Schémas des pages 8a et 8b :

Les équations 1, 3, 5 et 7 représentent la réaction des tétrahalocyclohexanols 1a, 1b, 1c et 1d pour former en présence de soude les trihaloépoxydes correspondants 2a, 2b, 2c et 2d. La régiosélectivité est totale lors des équations 5 et 7 et partielle pour l'équation 3 (l'époxyde α-fluoré 2b est obtenu avec son régiosomère α-chloré 2b' dans le rapport 2b/2b' = 13/1).
Les équations 2, 4, 6 et 8 représentent l'ouverture des trihaloépoxydes 2a, 2b, 2c et 2d par les réactifs HF/pyridine et/ou BF₃, Et₂O pour conduire sélectivement à l'introduction d'un atome de fluor en position cis par rapport à la fonction hydroxyle. Avec le réactif BF₃, Et₂O la diastéréosélectivité de la réaction est totale pour toutes les équations. Avec le réactif BF₃, Et₂O la diastéréosélectivité de la réaction est totale pour toutes les équations. Avec le réactif HF/pyridine cette diastéréosélectivité est encore totale pour les équations 2, 4, et 8 et de 5/1 (1d cis/ 1d'trans) pour l'équation 6.

### Schémas de la page 8c :

L' équation 9 représente une équation similaire à l'équation 1 dans laquelle on part de 2,2,6,6,-tétrachlorocyclohexanone sur laquelle on greffe un radical (içi figuré par R et qui correspond dans la formule générale à R₁) pour former l'alcool correspondant avec formation de l'époxyde dans les mêmes conditions que précédemment.
L'équation 10 représente l'ouverture de l'époxyde 2h tel qu'obtenu dans l'équation 9. Cette ouverture avec le réactif HF/pyridine se fait majoritairement avec l'atome de fluor en position cis du groupement hydroxyle. La diastéréosélectivité est dans le rapport 19 à 1 pour les fluorhydrines cis 1m /trans 1m'.
L'équation 11 montre la préparation des époxydes 2k, 2k', 2k'' à partir du mélange des alcools 1m, 1m' obtenu dans l'équation 10. Le produit secondaire 2k' est un composé provenant de l'alcool 1m.
Les équations 12 et 13 montrent comment on peut introduire un atome de fluor supplémentaire et aboutir après le traitement par la soude aux époxydes difluorés 21, 21'et 21'' en proportions inchangées par rapport aux mélanges des alcools 2k, 2k' et 2k''. L'époxyde majoritaire 21 qui contient 2 atomes de fluor sur le même carbone est en rapport de 9 à 1 vis des deux autres isomères.

### schémas de la page 8d :

Ces successions d'ouverture et de fermeture permettent de bien maîtriser, de manière très sélective, les substitutions des halogènes entre eux. Ceci est parfaitement exemplifié par l'inversion de la configuration montrée dans les équations de la scqsdfhéma III. Dans les équations 14-17, le problème est de faire un composé 1g difluoré comportant un fluor en position 6. Cette disposition permet de faciliter l'aromatisation pour donner des difluorobenzènes éventuellement substitués avec de meilleurs rendements qu'à partir du composé trifluoré 1d. La première réaction (équation 14) reprend l'équation 3. La seconde (équation 15) est celle de l'action du trichlorure de bore sur un mélange d'époxydes 2b, 2b'. Le produit majoritaire obtenu (équation 15) correspond à la structure du produit 1b obtenu lors de l'équation 2 de la schéma 1 mais dont la configuration a été inversée (hydroxyle et fluor en trans). Le traitement de 1f pur pour donner l'époxyde 2e (équation 16) puis son ouverture par le réactif BF₃, EtO₂ (équation 17) conduisent au produit 1g pur ayant en position 2 et 6, un chlore et un fluor.
L'équation 18 est un second exemple d'inversion de la configuration relative de la fluorhydrine 1g (possédant deux atomes de fluor portés par les carbones 2 et 6, l'un étant en cis l'autre en trans du groupement hydroxyle) en fluorhydrine 1h où les deux atomes de fluor sont en trans du groupement hydroxyle.

### schémas de la page 8e :

L'équation 19 montre comment on peut introduire par le tribromure de bore un atome de brome en cis de la fonction hydroxyle de l'alcool 1i, dont le traitement par la soude permet d'obtenir l'α-bromoépoxyde 2g.

Ainsi qu'il a été mentionné ci-dessus, la présente demande a également pour objet un procédé permettant l'aromatisation des cétones et des alcools selon la présente invention.

En ce qui concerne l'aromatisation des cétones, hydratée ou non, elle peut avoir lieu selon les procédés connus par l'homme de métier. Toutefois, selon la présente invention, il a été montré que le traitement par des noyaux aromatiques basiques tels que la pyridine et dérivés permettaient une obtention du noyau aromatique correspondant à la cétone.

Cependant, pour deshydrater les hydrates et lorsque l'aromatisation implique une deshyshydratation, pour en améliorer significativement la cinétique et le rendement, il était souhaitable d'utiliser des agents deshydratants tels que par exemple les tamis moléculaires dont le diamètre des pores est avantageusement inférieur à 1 nanomètre(10 Ä).

L'aromatisation de différentes cétones hydratées ainsi obtenues conduit au départ d'un groupement OH sur le carbone central (1) et un départ d'halogène sur chacun des carbones qui lui sont vicinaux.

L'aromatisation des cétones proprement dites peut être réalisée par action à chaud de systèmes de solvant(s) à caractère basique "lato sensu" tel que amines, sels d'amines, phosphines ou amides, associés eventuellement à des sels tels que des halogénures d'alcalins tels que LiCl ou de sels d'acides faibles tels que les carbonates.

Les halogènes qui partent, partent d'autant plus facilement que leur nombre atomique est plus élevé. Le départ des fluors est particulièrement difficile et nécessite un temps de réaction significativement plus élevé.

Ainsi les composés cétoniques selon la présente invention permettent d'obtenir aisément de manière spécifique, les phénols dihalogénés en 2 et en 6 avec de bons rendements.

Pour passer des alcools, selon la présente invention aux cétones correspondantes, on peut utiliser les techniques dites de Jones :
Bowden ; Heilbron ; Jones ; Weedon ; J. Chem. Soc. 39, 1946 et Bowers ; Halsall ; Jones ; Lemin ; J. Chem. Soc. 2548, 1953 ;
ou Dess-Martin : C.W. Perkins ; J.C. Martin ; A.J. Arduengo ; W. Lau ; A. Alegria ; J.K. Kochi. J. Am. Chem. Soc. 102, 7753, 1980 et D.B. Dess ; J.C. Martin ; J. Org. Chem. 48, 4155-56, 1953 ;
ou de Corey : Pyridinium chlorochromate (réactif de Corey) : Corey ; Suggs; Tetrahedron Lett. 2647, 1975.
Le schéma 5 représente la préparation des hydrates tétrahalogénés et leur déshydratation en cétones correspondantes.

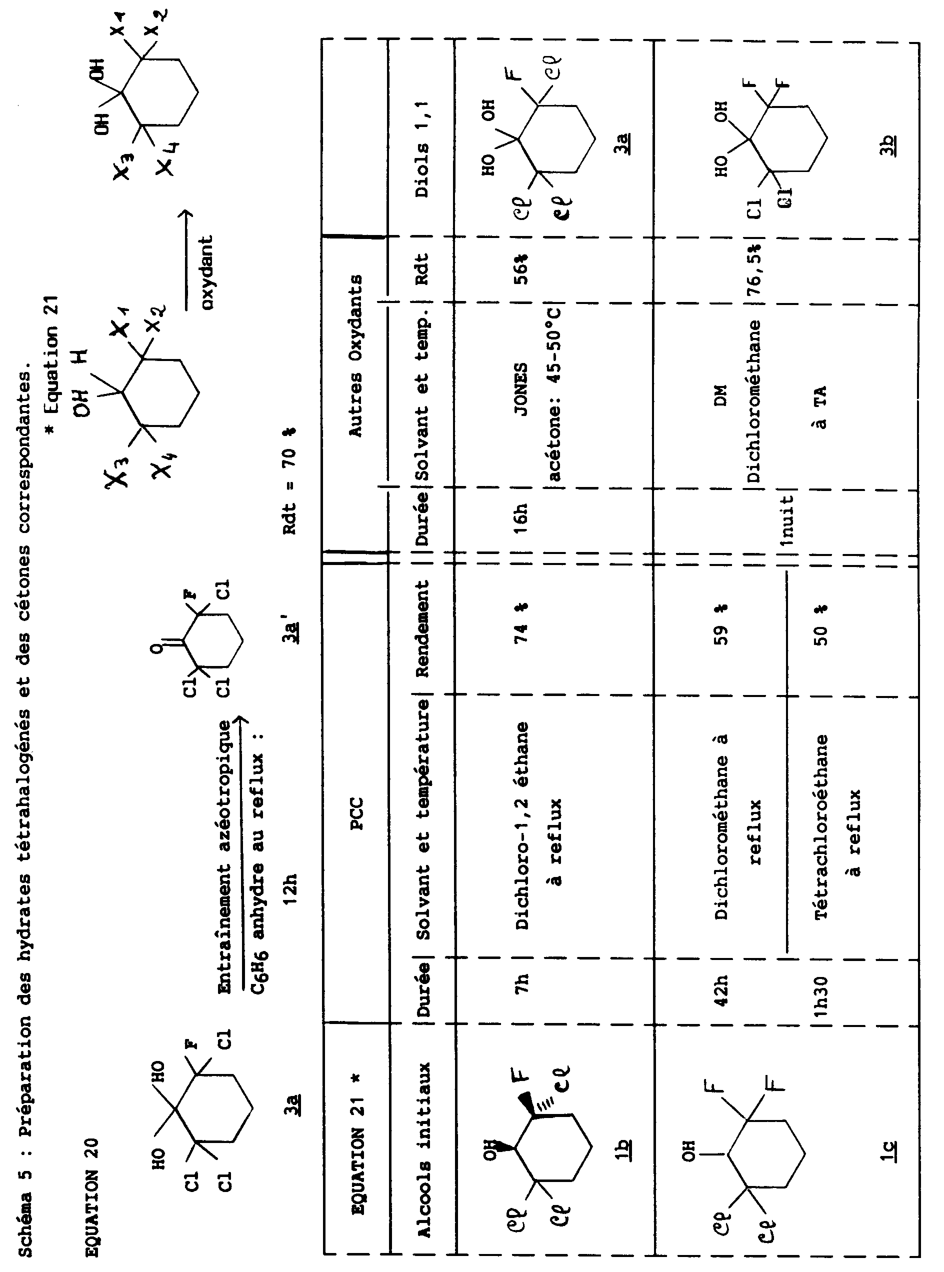

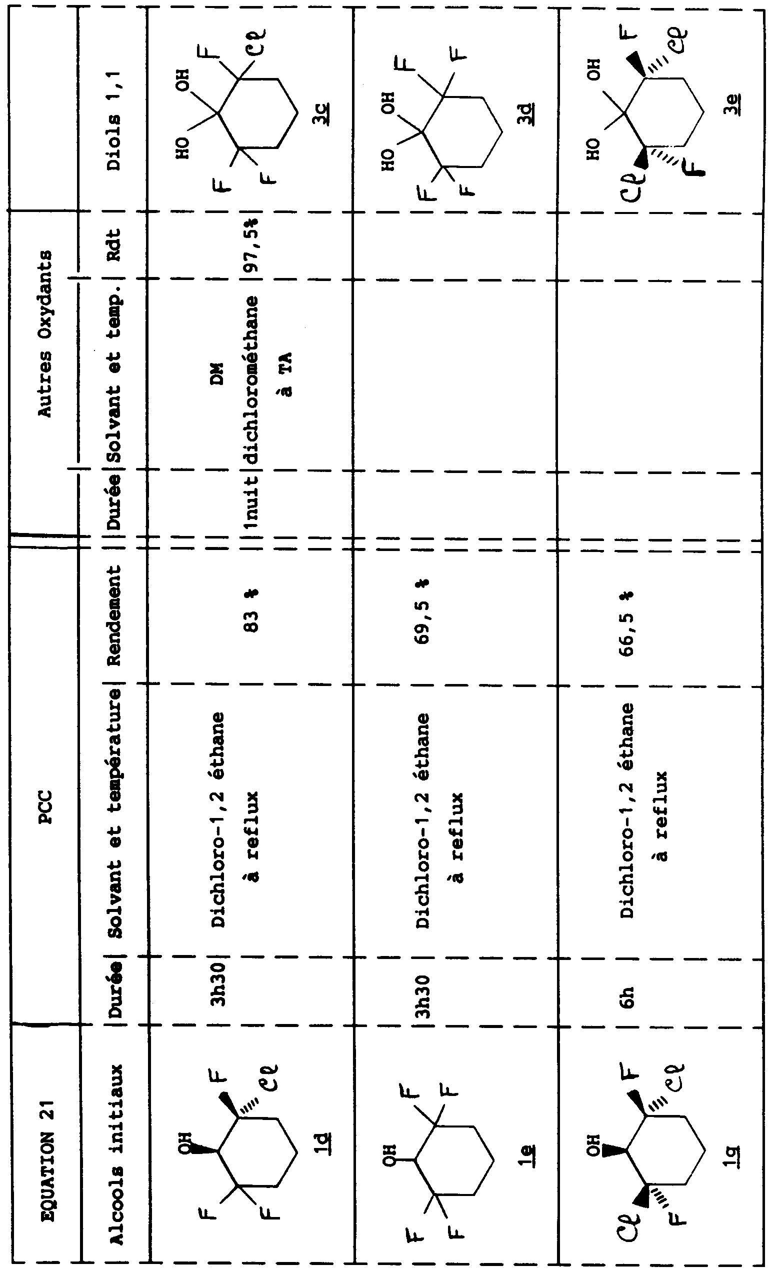

### Commentaires du schéma 5 :

### Schéma 5 :

Dans la schéma 5, l'équation 20 montre le passage de l'hydrate de cétone en cétone par entraînement azéotropique en présence d'un solvant aromatique, de préférence le benzène ou le toluène.

L'équation 21 représente les oxydations des fluorhydrines 1b-1e et 1g en cétones hydratées correspondantes 3a-3e à l'aide des réactifs pyridinium chlorochromate, Jones et Dess-Martin. Par cette méthode, l'hydrate de la 2,2,6,6-tétrafluorocyclohexanone 3d est obtenu avec un rendement de 69,5 %.

Les composés cétoniques peuvent être modifiés pour donner, naissance à des intermédiaires réactionnels qui sont aromatisables comme les cétones, on peut ainsi passer d'un composé cétonique à un équivalent comme par exemple les thiocétones, les acétals, les imines, les oximes les doubles liaison par actions sur les méthylènes acides (par exemple par synthèse de type malonique).

Les cétones selon l'invention peuvent réagir sur les phénols pour donner des composés de mono- ou de bis-substitution, composés intéressants même sans qu'il soit nécessaire d'aromatiser.

Les réactions de réduction (par exemple LiAlH₄ dans le THF pendant une nuit à reflux) sur les imines permettent d'obtenir des aziridines qui sont des intermédiaires de synthèses particulièrement interressant. ainsi dans les composés de la formule II ont comme équivalents simples les composés où l'azote se substitue à l'oxygène.

L'oxydation d'un l'alcool selon l'invention, suivie de sa réduction est envisageable pour permettre d'obtenir un alcool ou un mélange d'alcool de configuration différente de celle de départ. Il est également possible d'aromatiser directement les alcools pour donner un phénol substitué en ortho.

Ainsi qu'il a été mentionné ci-dessus, la présente demande a également pour objet un procédé permettant l'aromatisation les époxydes selon la présente invention pour donner un phénol méta substitué.

Les conditions sont analogues à celles de l'aromatisation des alcools ou des cétones ; elles sont avantageusement les suivantes : chauffage à reflux dans des bases aminées ou des amides ; Il est à noter cependant que lorsque les amines présentent un hydrogène substituable certaines réactions autres ou en sus de l'aromatisation peuvent avoir lieu, notamment le remplacement d'un halogène par ladite amine.

Les amides sont toutefois préférées. Cette aromatisation permet d'obtenir des composés substitués en méta d'une fonction phénol, c'est-à-dire que l'aromatisation conduit à une fonction phénol sur l'un des carbones vicinaux (c'est-à-dire 2 ou 6) portant une des jambes du pont époxyde. L'élimination de l'halogène sur l'autre carbone vicinal se fait dans les règles déjà mentionnées, c'est-à-dire que reste l'halogène dont l'halogénure fait le plus mauvais groupe partant. On peut également obtenir un phénol lorsque le carbone 1 de l'époxyde de formule (II), porte une chaîne hydrocarbonée. On obtient dans ces conditions un phénol substitué en ortho par la chaîne hydrocarbonée et substituée en méta par un halogène.

Le schéma 6 illustre les réactions d'aromatisation permettant l'obtention de dérivés du phénol mono et disubstitués régiosélectivement.

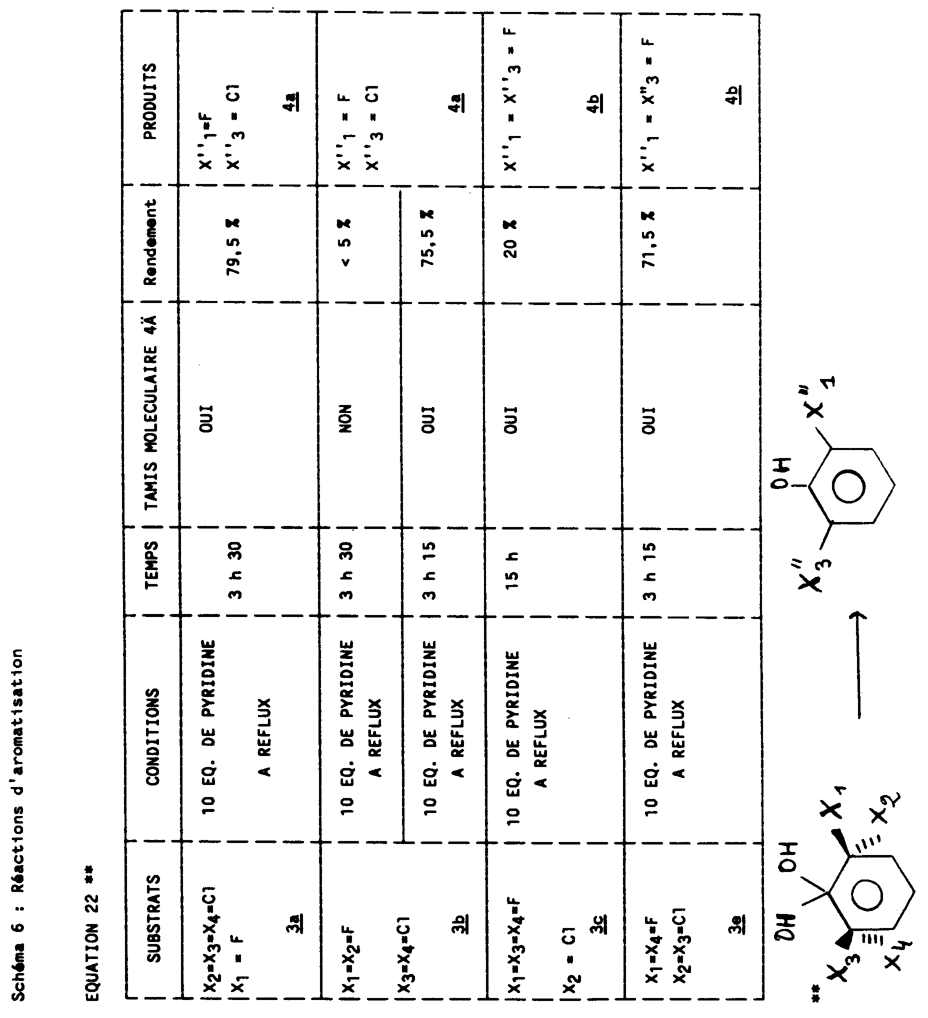

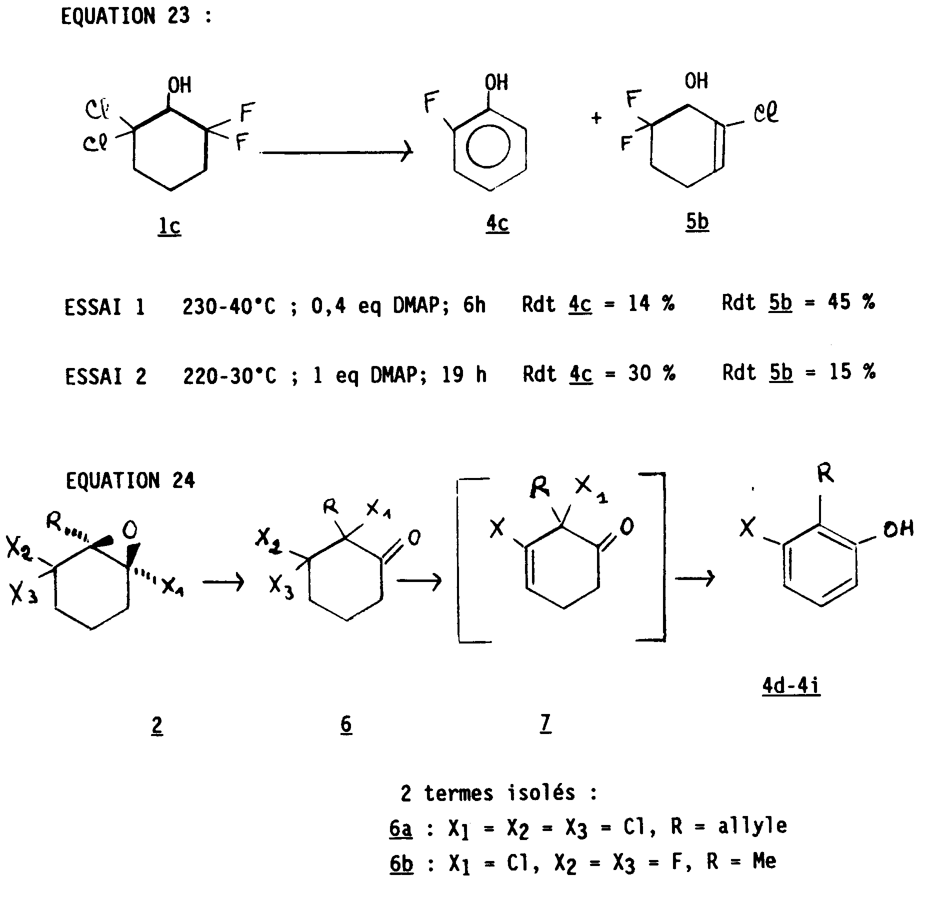

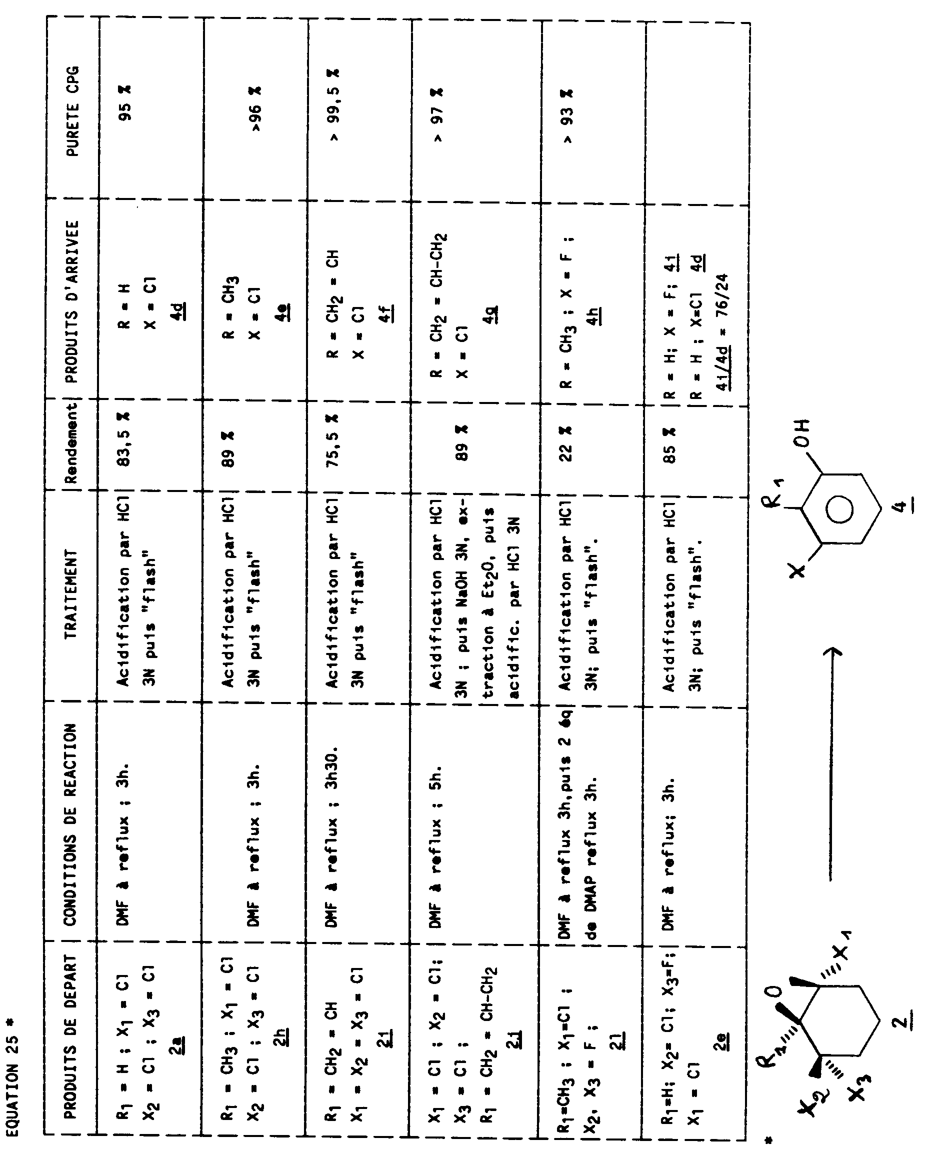

### Schéma 6 :

L'équation 22 présente la transformation des hydrates de cétones tétrahalogénés 3a-c et 3e en phénols 2,6-dihalogénés 4a-4b. Cette réaction doit être effectuée en présence d'un déhydratant de préférence des tamis moléculaires 4Ä.
L'équation 23 montre l'aromatisation de la fluorhydrine 1c en 2-fluorophénol 4c. Dans cette réaction la formation du 2-fluorophénol 4c est accompagnée de l'alcool allylique 5b.
L'équation 24 montre la formation intermédiaire de la cétone 2,3,3-trihalogénée 6 dans la réaction d'aromatisation des époxydes 2 (R=alkyle). La cétone dihalogénée β γ-insaturée 7 est postulée comme étape intermédaire pour l'accès aux phénols 4. Les phénols 4d-4i métahalogénés et alkylés en position 2 (ou porteur d'un hydrogène dans cette même position) sont décrits dans l'équation 25 et obtenus par chauffage dans la diméthylformamide des époxydes correspondants.
Les exemples non limitatifs suivants illustrent l'invention.

### Préparation du trichloro-1,3,3-oxa-7-bicyclo [4.1.0] heptane 2a

A 15,2 g (63,88 mmol) de tétrachloro-2,2,6,6-cyclohexanol 1a en suspension dans 70 cm³ d'eau distillée, on ajoute à 10-15° C en trois fois 7g ( 175 mmol) de soude en perle. On maintient le mélange réactionnel 1h15 à température ambiante, ajoute ensuite 20 cm³ d'éther éthylique, laisse l'agitation pendant 5 mn puis on décante et on extrait la phase aqueuse à l'éther (6 x 50 cm³). On regroupe les phases éthérées, on sèche sur MgSO₄, filtre et concentre le mélange avec un évaporateur rotatif. On obtient un liquide, l'époxyde 2a (m= 12,42 g) représentant 96,5 % du rendement théorique. Le composé 2a sera souvent utilisé sans purification ultérieure dans les étapes suivantes (pureté en CPG semi-capillaire > 96 %). L'époxyde 2a peut être purifié par distillation sous pression réduite (ébullition à 40°C sous 0,1 mmHg); le rendement après distillation en composé 2a est de 92 %.
Eb : 213°C
RMN ¹H (CDCl₃ + 3 % TMS) : 1,25-2,0 (m, 2 H);
2,0-2,8 (m, 4 H); 3,8 (s, 1 H).
RMN ¹³C (CDCl₃) : 84,37 (s,C₃); 73,36 (s,C₁); 65,50 (d, C₂) 38,70 (t, C₄); 31,49 (t, C₆); 18,51 (t, C₅)
PM : 201,479
IR en cm⁻¹ (pur) : 1080 - 1130

| Microanalyse : C₆H₇Cl₃O | | | | |
|---|---|---|---|---|
| Calc. % | C | 35,77 | H | 3,50 |
| Tr. % | | 35,83 | | 3,57 |

### Préparation du dichloro-3,3-fluoro-1 oxa-7-bicyclo [4.1.0] heptane 2b

Le même mode opératoire est appliqué à 2g (9,03 mmol) d'alcool 1b en suspension dans 12 cm³ d'eau distillée et 1,5g ( 37,5 mmol) de soude. Temps de réaction 1h15 extraction à l'éther (5 x 25 cm³). On obtient 1,61g de produit brut dont l'analyse en RMN¹H (80 MHz) laisse apparaître la présence des époxydes 2b et 2b' en proportion 2b/2b' = 93/7 (calcul par intégration des signaux à 3,88 et 3,82 ppm représentant le proton porté par le carbone n°2 de l'époxyde 2b et de son régioisomère 2b'). Le rendement brut est de 96 % ; l'époxyde 2b sera utilisé tel quel du fait de l'impossibilité de le séparer de 2b' facilement. RMN¹H (CDCl₃) : 3,93 (d, 1H, J_{HF} = 1,8 Hz); 2,47-1,38 (m, 6H).
- IR (film) en cm⁻¹ :: 1180, 1085, 1040, 930, 890, 820, 770.
- RMN¹³C (C₆D₆):: 103, 52-89,66 (d, C₁, J₁ = 277, 2 Hz); 84,97 (C₃); 63,35-62,88 (d, C₂, J₂ = 10,5 Hz); 39,20 (C₄); 24,94-23,62 (d, C₆, J₂ = 25,9 Hz); 17,79-17,37 (d, C₅, J₃ = 8,4 Hz).

### Préparation du chloro-1-difluoro-3,3-oxa-7-bicyclo [4.1.0] heptane 2c

Le même mode opératoire est appliqué à 0,6 g (2,93 mmol) d'alcool 1c pur en suspension dans 5 cm³ d'eau distillée et 0,48 g ( ≃ 12 mmol) de soude. Temps de réaction 1h15. Extraction à l'éther (5 x 15 cm³). On obtient 0,43 g d'un liquide, l'époxyde 2c, soit un rendement brut de 87 %.
RMN¹H (CDCl₃) : 3,56 (t, 1H, J = 2,2 Hz); 2,79-1,32 (m, 6H).
RMN¹³C (C₆D₆) : 131,9-120,1-108,2 (t,C₃), 65,8 (C₁);
61,18-59,17-57,13 (t,C₂); 32,42 (C₆);
30,81-29,66-28,47 (t, C₄); 17,09-16,87-16,06 (t, C₅).
IR (film) en cm⁻¹ : 1120, 1100, 975, 835.

| Microanalyse C₆H₇ClF₂O | | | | |
|---|---|---|---|---|
| tr : | %C | 43,40 | %H | 4,19 |
| th : | | 42,75 | | 4,19. |

### Préparation du trifluoro-1,3,3-oxa-7-bicyclo [4.1.0] heptane 2d

Même mode opératoire effectué avec 0,72 g(3,8 mmol) d'alcool trifluoré 1d pur, avec 5 cm³ d'eau distillée et 0,55 g (≃ 13,7 mmol) de soude. Temps de réaction 1h15. Extraction à l'éther (5x15 cm³); on obtient 0,36 g d'époxyde 2d soit un rendement de 62 % brut.
- RMN¹H (CDCl₃): : 3,61 (q, 1H, J = 1,9 Hz) ; 2,40-1,45 (m, 6H).
- IR en cm⁻¹ (CDCl₃) :: 1460-1370-1250-1240-1210.

### Préparation du trichloro-2,2,6-fluoro-6-cyclohexanol (cis) 1b

### - Par HF/pyridine

Dans un flacon de polyéthylène muni d'un barreau magnétique, d'un septum (milieu réactionnel possédant une sortie vers la hotte aspirante), on introduit 3,2 cm³ de HF/pyridine (à la seringue en polyéthylène). Le milieu réactionnel est porté vers -35°C (température du bain extérieur) et on additionne en 25 mn grâce à un pousse-seringue 1,93 g (9,6 mmol) d'époxyde 2a. On laisse ensuite réagir 3H à la même température, puis remonte à température ambiante 30 mn. On hydrolyse par de la glace à 0°C, extrait au dichlorométhane (5x20 cm³), sèche sur sulfate de magnesium, filtre, évapore les solvants. On recueille 2,03 g d'une huile composée de 85 % d'alcool fluoré 1b et 15 % d'alcool allylique 5a (calcul par RMN¹H: intégration du proton au pied de la fonction alcool du composé 1b (d; 4,04 ppm, J₁ = 18,4Hz) par rapport au proton au pied de la fonction alcool du composé 5a (s;4,36 ppm). Les deux alcools 1b et 5a n'étant pas séparables par distillation ou chromatographie éclair sur silice, nous avons envisagé une purification chimique de l'alcool 1b. Après cette purification , le rendement sur composé 1b pur est de 82 % par rapport à l'époxyde de départ 2a.

### Par BF₃, Et₂O

Dans un ballon de 25 cm³, on a introduit 1g (4,96 mmol) d'époxyde 2a préalablement distillé en solution dans 7 cm³ de dichlorométhane. On ajoute 0,325 cm³ (0,53 eq) de BF₃, Et₂O et on porte à reflux pendant 3h (la fin de la réaction est suivie en CPG). On hydrolyse par HCl 6N (10 cm³) pendant une nuit, extrait au dichlorométhane (5x30 cm³), sèche sur sulfate de magnésium et évapore les solvants. On incorpore l'huile obtenue sur silice et purifie par chromatographie éclair (éluant : éther/éther de pétrole = 10/90); on obtient 1,01g d'un solide blanc, l'alcool monofluoré 1b. Le rendement de la réaction est de 92 %.
F = 37-38° C
RMN¹H (CDCl₃) : 4,04 (dd, 1H, J_{HF} = 18,4 Hz, J_{H-OH} = 9,6 Hz); 3,14 (d, 1H mobile, J = 9,6 Hz); 2,96-1,49 (m, 6H).
RMN¹³C (C₆D₆) : 118,78-106,06 (d, C₆, J_{CF} = 254, 5 Hz); 91,62-91,52 (d, C₂, J_{CF} = 1,9 Hz); 81,73-80,78 (d, C₁,
J_{CF} = 19 Hz); 44,67 (s, C₃); 40,08-38,96 (d, C₅, J_{CF} = 22,3 Hz); 19,59 (s, C₄).
IR (en cm⁻¹) (CDCl₃) : v _{OH} = 3570

| Microanalyse : C₆H₈ OCl₃F | | | | |
|---|---|---|---|---|
| tr. | %C : | 32,59 | %H: | 3,52 |
| th. | | 32,54 | | 3,64 |

### Caractéristique de l'alcool allylique 5a

- RMN¹H (CDCl₃) :: 5,97 (t, 1H); 4,36 (s, 1H). 3,1 (s, 1H mobile); 2,9-2,1 (m, 4H).
- RMN¹³C (CDCl₃) :: 128,20 (s, C₂); 126,69 (d,C₃); (couplage C-H) 90,98 (s, C₆); 76,90 (d, C₁); 35,43 (t,C₅); 25,06 (t,C₄).
- IR (CDCl₃) en cm⁻¹ :: v_{OH} = 3580 (bande fine); _{C=C} = 1650.

### Préparation du dichloro-2,2-difluoro-6,6-cyclohexanol 1c

### Par HF/pyridine

Dans un flacon de 20 cm³ en polyéthylène, sous agitation magnétique, muni d'un septum et d'une sortie vers la hotte aspirante, on a introduit 2,75cm³ de complexe HF/pyridine. On additionne à 0°C en 20 mn 1,53 g (8,27 mmol) d'époxyde 2b (contenant 7 % du régioisomère 2b'), on laisse réagir 10 mn puis on remonte à température ambiante et on poursuit l'agitation pendant 50 mn. On ajoute à -10°C 10 cm³ d'eau distillée, extrait au dichlorométhane (5x20 cm³), on sèche sur sulfate de magnésium, on filtre et évapore pour obtenir 1,67 g de produit brut dont l'analyse en CPG semi-capillaire laisse apparaître la présence de deux régioisimères 1c et 1c' en proportion 93/7. La purification peut être réalisée de deux façons différentes :
- Par chromatographie éclair sur silice (séparation possible de 1c et 1c'): Dans un premier essai, on a isolé le mélange des alcools 1c et 1c' avec un rendement de 86 % (éluant : éther/éther de pétrole = 90/10).
   Dans un second essai on a modifié les conditions de séparation (éluant : éther/éther de pétrole = 96/4) et ainsi obtenu 1,2 g (Rdt = 71 %) d'alcool 1c, (pureté en CPG semi-capillaire > 98,5 %) et 0,2 g (Rdt = 12 %) des alcools 1c et 1c' en proportion 82/18.
   Dans le but de caractériser sans ambiguité la structure de l'alcool 1c, on a augmenté les quantités de produit de départ ( ≃ 10g d'époxyde 2b) et ainsi pu isoler en plus de l'alcool 1c 200 mg d'alcool 1c' d'une pureté en CPG semi-capillaire > 98,5 % après trois chromatographies éclairs sur silice.

### Par BF₃, Et₂O

Même mode opératoire que pour 1b : effectué sur 1,35 g des époxydes 2b et 2b' (2b/2b' = 93/7) en solution dans 10 cm³ de dichloro-1,2 éthane et 0,26 cm³ (0,55 eq) de BF₃, Et₂O ; durée du reflux 1h45. On hydrolyse par 10 cm³ d'eau pendant une nuit. La purification s'effectue comme précédemment (colonne O = 2cm, h = 9 cm, éluant éther/éther de pétrole = 10/90). On obtient 1,23 g d'un liquide incolore, l'alcool difluoré 1c (en mélange avec 7 % d'alcool 1c'). Le rendement global de la réaction est de 82 %.
Alcool 1c
RMN¹H (CDCl₃) : 3,99 (dd, 1H, J₁ = 15,95 Hz, J₂ = 6,4 Hz): 3,14 (s, 1H mobile); 2,93-1,18 (m, 6H).
RMN¹³C (C₆D₆): 132,85 - 120,54 - 108,07 (t, C₆); 91,35-90,98 (d,C₂, J₃ = 7,4 Hz ); 78,35 - 77,73 - 76,46 (dd, C₁); 42,84 (C₃); 32,43-31,29 - 30,13 (t, C₅); 18,75 - 18,67 (d, C₄, J₃ = 1,6 Hz).
IR (pur) en cm⁻¹ : v _{OH} = 3550 (fine); 3450 (large).

| Microanalyse C₆H₈Cl₂F₂O | | | | |
|---|---|---|---|---|
| tr. | %C | 34,94 | %H | 3,86 |
| th. | | 35,15 | | 3,93 |

Alcool 1c'
F = 80-81°C
RMN¹H (CDCl₃) : 3,93 (dt, 1H, J₁ = 20,6 Hz, J₂ = 9,6 Hz); 2,93 (d, 1H mobile, J= 9,6 Hz); 2,85-1,45 (m, 6H).
RMN¹³C (CDCl₃) : 111,64 (d, C₁, J = 254,3 Hz); 79,50 (t, C₂ et C₆, J = 19,7 Hz) ; 39,46 (t, C₃ et C₅, J= 11,3 ± 0,5 Hz) ; 18,58 (S, C₄).
IR (CDCl₃) en cm⁻¹ : v_{OH} = 3450 cm⁻¹ (large).

| Microanalyse : C₆H₈Cl₂F₂O | | | | |
|---|---|---|---|---|
| tr. | %C | 34,98 | %H | 3,82 |
| th. | %C | 35,15 | %H | 3,93 |

### Préparation du chloro-2-trifluoro-2,6,6-cyclohexanol 1d

### Par HF/pyridine

Dans une bouteille de polyéthylène de 10 cm³, munie d'un septum, d'une sortie vers la hotte aspirante et sous agitation magnétique, on introduit 3 cm³ de complexe HF/pyridine. On additionne en 25 mn à 0°C 1,23 g (7,30 mmol) d'époxyde chloré 2c, on laisse réagir 10 mn puis on remonte à température ambiante et poursuit l'agitation pendant 1h20.
On ajoute à 0°C 7 cm³, extrait au dichlorométhane (5x15 cm³), sèche sur sulfate de magnésium, filtre et évapore le solvant. On purifie par chromatographie éclair sur silice le produit brut obtenu (1,25 g); colonne : 0 = 2,8 cm, h = 13 cm, éluant : éther/éther de pétrole = 10/90 à 12/88. On isole 0,16 g (0,85 mmol) du diastéréoisimère 1d pur, 0,8g (4,24 mmol) de mélange 1d et 1d' avec 1d/1d' = 95/5 (déterminé par CPG semi-capillaire) et 0,19 g (1,01 mmol) du diastéréoisomère 1d' pur. Le rendement global de la réaction est de 83,5 %, les proportions 1d/1d' globales (en produits isolés) sont d'environ 83/17.

### Par Bf₃, Et₂O

Même mode opératoire que pour 1b : effectué sur 0,39g (2,31 mmol) d'époxyde 2c en solution dans 3,3 cm³ de dichlorométhane et 0,16 cm³ (0,55 eq) de BF₃, Et₂O; durée de reflux : 1h30. On hydrolyse par 5 cm³ d'HCl 6N pendant une nuit. La purification s'effectue comme précédemment (éluant : éther/éther de pétrole = 10/90) et permet d'isoler 0,36g d'alcool trifluoré 1d ne contenant pas, d'après la CPG semi-capillaire d'isomère 1d'. Le rendement de la réaction est de 82,5 %. Alcool 1d
F°C = 42-43.
RMN¹H (CDCl₃) : 3,98 (d,t, 1H, J = 16,6 Hz, J= 5,1 Hz); 3,10 (s, 1H mobile) 2,80-1,50 (m, 6H).
RMN¹³C (C₆D₆) : 120,95 (t, C₆, J = 246,6 Hz); 94,6 (d, C₂, J = 246,6 Hz); 77,49 - 76,52 - 75,18 - 74,21 - (q,C₁); 37,61 (d, C₃, J =22 Hz); 31,36 (t, C₅, J= 22 Hz); 17,52 (s,C₄).
IR en cm⁻¹ (film) : v_{OH} = 3400.

| Microanalyse : C₆H ClF₃O. Alcool 1d' | | | | |
|---|---|---|---|---|
| th. | %C | 33,21 | % H | 4,28 |
| tr. | | 38,30 | | 4,11 |

RMN¹H (CDCl₃) : 4,22-3,78 (m, 1H); 2,75 (d, 1H mobile, J = 6 Hz) 2,65-1,53 (m, 6H).
IR (pur) en cm⁻¹ : v _{OH} = 3440 cm⁻¹ (large).
RMN¹³C (CDCl₃) : 122,71 (t,C₆, J = 232 ± 8 Hz); 114,73 (d, C₂,J= 246,6Hz) 75,94 ; 74,60; 73,27; 71,93 (q, C₁) ; 35,99 (d, C₃, J = 19,53 Hz); 29,44 (t, C₅, J = 23 ± 1 Hz); 17,55 (t,C₄, J = 4,88 Hz).

### Tétrafluoro-2,2,6,6-cyclohexanol 1e

Même mode opératoire : effectué avec 0,3 cm³ de complexe HF/pyridine pour 0,09 g (0,59 mmol) d'époxyde 2d en solution dans 0,1 ml de THF (addition au pousse seringue en 10 mn à 0°C puis laisse remonter à température ambiante 1h). On ajoute 3 cm³ d'eau distillée à 0°C, extrait au dichlorométhane (3x 10cm³).
Après chromatographie éclair sur silice (éluant : éther / éther de pétrole = 30/70), on isole 0,05 g (0, 29 mmol) d'un solide blanc, l'alcool 1e avec un rendement de 49 %.
- F°C = 65-66.:
- RMN¹ H (CDCl₃) 400 MHz :: 4,00 - 3,85 (m, 1H); 2,61 (d, 1H mobile, J = 1Hz); 2,25-2,05 (m, 2H); 1,98-1,78 (m, 2H); 1,78-1,63 (m, 2H).
- IR en cm⁻¹ (CDCl₃) :: v _{OH} = 3590
- RMN¹³C (CDCl₃) 100 MHz :: 120,89 (t, C₂ et C₆, J = 248 Hz); 71,72 (m, C₁) 29,96 (t,C₃ et C₅, J = 22,5 Hz); 16,18 (d, C₄, J = 5,4 Hz).

### Préparation du trichloro-2,2,6-fluoro-6 cyclohexane-1,1-diol 3a

### Par le PCC

Dans un ballon de 25 cm³, muni d'un réfrigérant et sous agitation magnétique, on a introduit 0,96 g (4,33 mmol) d'alcool trichloré fluoré 1b en solution dans 10 cm³ de dichloroéthane. On ajoute à la spatule 2,33 g (2,5 eq) de PCC et on porte à reflux sous atmosphère d'argon pendant 7h. On laisse refroidir le milieu réactionnel que l'on reprend ensuite par 100 cm³ d'éther, le tout étant filtré sur florisil. On évapore les solvants, incorpore sur silice et purifie par chromatographie éclair (éluant : éther/éther de pétrole = 15/85 puis 20/80). On isole 0,03 g d'alcool 1b de départ, soit environ 3 %, 0,05g de mélange alcool 1b diol 3a et 0,76 g de diol 3a pur. Le rendement en composé est donc supérieur à 74 %.

### Par le réactif de Jones

La composition du réactif de Jones est la suivante : 6,7 g de CO₃; 14,5 ml d'eau distillée ; 5,8 ml de H₂SO₄ à 98 %.
Dans un ballon de 10 cm³, muni d'un réfrigérant et d'une agitation magnétique, on a introduit 0,5 g (2,26 mmol) d'alcool 1b en solution dans 3 cm³ d'acétone. On introduit doucement 5 cm³ du réactif de Jones, la réaction est fortement exothermique ; on porte ensuite le milieu réactionnel à 45-50°C pendant 16 h (en ayant rajouté 1 cm³ de réactif de Jones au bout de 4h et 2 cm³ après 9h de réaction). On détruit en fin de réaction le réactif de Jones par 10 cm³ d'isopropanol, dilue le milieu avec 10 cm³ d'eau et extrait au dichlorométhane, sèche sur sulfate de magnésium et évapore les solvants. On incorpore sur silice et purifie en chromatographie éclair. On isole 0,16 g d'alcool 1b de départ (32 %) et 0,30 g de diol 3a pur, soit un rendement en composé 3a de 56 % (Rdt basé sur l'alcool 1b non récupéré = 85 %).
F° = 42-43°C.
RMN¹H (CDCl₃): 3,75 (s, 1H mobile); 3,60 (s, 1H mobile); 2,70-1,40 (m,6H).
IR en cm⁻¹ (CDCl₃) : v _{OH} = 3540 (très large).

| Microanalyse : C₆H₈Cl₃FO | | |
|---|---|---|
| | % C | % H |
| th. : | 30,34 | 3,39 |
| tr. : | 30,26 | 3,15 |

### Préparation du dichloro-2,2-difluoro-6,6-cyclohexane-1,1-diol 3b

1er essai : Dans un tricol de 50 cm³, muni d'un thermomètre, d'un réfrigérant et sous agitation magnétique, on a introduit 0,5 g (2,44 mmol) d'alcool 1c en solution dans 20 ml de dichlorométhane. On ajoute 1,76g (16,42 mmol) de sulfate de magnésium et 2,10 g (9,75 mmol) de chlorochromate de pyridinium en maintenant une vive agitation. On porte à reflux sous atmosphère d'argon pendant 42 h. La fin de la réaction est contrôlée par chromatographie sur couche mince. Le milieu réactionnel est alors repris à l'éther et filtré sur florisil, on évapore alors les solvants et purifie le produit brut par chromatographie éclair sur silice. On isole 0,32 g (1,44 mmol) de diol, 3b (Rdt = 59 %) distillable et fondant vers 30°C. Notons que le composé 3b est sublimé au dessicateur.
2ème essai : On procède de la même façon avec le tétrachloroéthane ; on porte à reflux ( 127°C) pendant 1h30, temps auquel on ne constate plus la présence d'alcool 1c en chromatographie sur couche mince. On filtre sur florisil en rinçant à l'éther et évapore. Le produit brut est distillé sous vide partiel (trompe à eau). La première fraction est le tétrachloroéthane (eb°C = 45/13 mmHg). On isole ensuite le diol 3b par chromatographie élair sur silice (éluant : éther / éther de pétrole = 15/85 pour éliminer le tétrachloroéthane restant puis 25/75 pour obtenir le diol 3b). On obtient un rendement de 50 % en diol 3b.

### Oxydation par le périodinane (réactif de Dess-Martin) :

A 1,14 g (5,56 mmol) d'alcool 1c en solution dans 20 ml de dichlorométhane, on ajoute 6,2 g (≃ 2,5 eq) de réactif de Dess-Martin. On laisse le milieu réactionnel sous agitation magnétique pendant une nuit à température ambiante. On ajoute ensuite 100 cm³ d'éther puis verse dans 150 cm³ d'une solution 0,52 M de Na₂S₂O₃ et saturée en NaHCO₃ ; on décante, sépare la phase organique de la phase aqueuse qui est extraite de nouveau par 2 fois 25 cm³ d'éther. On sèche sur MgSO₄ puis évapore. On obtient après filtration sur silice 0,94 g de diol 3b soit un rendement de 76,5 %.
- F = 30°C:
- RMN¹H (CDCl₃) :: 3,75 (s, 2H mobile) ; 2,62-1,71 (m, 6H).
- RMN¹³C (CDCb) :: 119,85 (t, C₆, J = 253,91 Hz) ; 93,39 (t,C₁, J = 24,41 Hz); 92,90 (s, C₂) ; 41,91 (s, C₃); 29,77 (t,C₅, J = 21,97 Hz) ; 17,75 (t,C₄, J=4,88 Hz).
- IR en cm-¹ (film) :: v OH = 3700-3000 (v _{C=O}= 1770)

### Préparation du chloro-2, trifluoro-2,6,6-cyclohexane-1,1-diol 3c

### Par le PCC.

Même mode opératoire que 3a ; effectué avec 0,60 g (3,18 mmol) d'alcools trifluorés 1d et 1d'(en proportion 1d/1d' = 95/5) en solution dans 10cm³ de dichloroéthane et 1,90 (2,77 eq.) de PCC. On porte à reflux pendant 3h30 ; on obtient après traitement sur florisil 0,54 g (2,62 mmol) de diol 3c brut, soit un rendement en composé 3c de 83 %.

### Par le réactif de Dess-Martin

A 1,02 g (5,41 mmol) d'alcool 1d en solution dans 20 cm³ de dichlorométhane on ajoute à température ambiante 6g ( 2,5 eq) de réactif de Dess-Martin. On abandonne le milieu réactionnel à température ambiante pendant 1 nuit. On ajoute ensuite 100 cm³ d'éther et verse le tout dans 150 cm³ d'une solution 0,6 M de Na₂S₂O₃ et saturée en NaHCO₃. On décante, sépare les phases éthérées et aqueuses, extrait de nouveau avec 3x20 cm³ d'éther. On regroupe les phases organiques et après séchage sur MgSO₄ évapore les solvants. On obtient alors 1,08 g de diol 3c brut, soit un rendement de 97,5 %.
- F°C = 35-36.:
- RMN¹H (CDCl₃) :: 3,66 (s, 2H mobiles) ; 2,65-1,50 (m, 6H).
- IR en cm⁻¹ (CDCl₃) :: v _{OH} = 3560 (très large).

### Préparation du tétrafluoro-2,2,6,6-cyclohexane-1,1-diol 3d

### Par le PCC

Même mode opératoire que 3a ; effectué sur 0,04 g (0,23 mmol) d'alcool tétrafluoré 1e en solution dans 3 cm³ de dichloroéthane et 0,20 g (0,92 mmol) de PCC. On porte 3h30 à reflux et obtient après traitement sur florisil puis lavage au pentane 0,03 g (0,16 mmol) d'un solide blanc, le diol tétrafluoré 3d.
Le rendement en produit 3d est de 69,5 %.
F°C = 107,108.
RMN¹H (CDCl₃) : 3,41 (s, 2H mobiles); 2,60-1,45 (m, 6H) ;
IR (CDCl₃) en cm⁻¹ : _{OH} = 3570 (large).
PM = 188,123.
RMN¹³C (CDCl₃) 100 MHz : 119,97 (t, C₂ _{et C6}, J = 252,15 ± 0,75 Hz) ; 91,36 (t, C₁, J= 26,32 Hz); 29,8 (m, C³ et C₅) ; 15,5 (s, C₄).

| Microanalyse : C₆H₈F₄O₂ | | | | |
|---|---|---|---|---|
| th. | %C | 38,31 | % H | 4,29 |
| tr. | | 38,05 | | 4,24. |

### Préparation du chloro-2-fluoro-6-phénol 4a

### A partir du diol 3a

Dans un ballon de 10 cm³, muni d'un réfrigérant, on porte à reflux pendant 3h20 sous atmosphère d'argon 0,51 g (2,15 mmol) de diol 3a en solution dans 1,65 cm³ ( ≃ 10 eq) de pyridine en présence de 1,4 g de tamis moléculaire 4Ä. On acidifie à Ph 1 en fin de réaction avec 10 cm³ d'acide chlorhydrique 6N, extrait à l'éther (5x20 cm³), sèche sur sulfate de magnésium, filtre et évapore le solvant. On obtient un solide brut que l'on purifie par chromatographie éclair sur silice (éluant : éther/éther de pentane = 10/90 ). On obtient 0,25g (1,7 mmol) d'un solide blanc à forte odeur phénolique, le 2-chloro-6 fluorophénol 4a Rdt = 79,5 %.

### A partir du diol 3b

Dans un ballon de 10 cm³, on chauffe à reflux et sous atmosphère d'argon pendant 3h15 0,34 g (1,54 mmol) de diol 3b en solution dans 1,2 cm³ (10 eq) de pyridine en présence de 1g de tamis moléculaire 4Ä. On contrôle la réaction par CPG capillaire. On acidifie en fin de réaction par 8 cm³ d'acide chlorhydrique 4N, extrait au dichlorométhane (5x10 cm³), sèche sur sulfate magnésium, évapore le solvant.
On obtient 0,19 g d'une huile brute que l'on purifie par chromatographie éclair sur silice (éluant : éther/pentane = 10/90). On recueille 0,17g (1,16 mmol) d'un solide blanc, le 2-chloro-6-fluorophénol 4a (Rdt = 75,5%).
F°C = 64.
RMN¹H (CDCl₃) : 7,30-6,60 (m, 3H) ; 5,55 (s, 1H mobile).
IR en cm⁻¹ (CDCl₃) : v _{OH} = 3540 v _{C=C} = 1600.

| Microanalyse : C₆H₄ClFO | | | | |
|---|---|---|---|---|
| th. : | %C | 49,18 | %H | 2,75 |
| tr. : | | 48,95 | | 2,57 |

### 2) 2,6-difluorophénol 4b

### a) A partir du diol 3c

Même opératoire que ci-dessus : effectué sur 0,40 g (1,94 mmol) de diol trifluoré 3c, 10 équivalents de pyridine (soit environ 1,56 cm³), 1g de tamis moléculaire 4Ä ; reflux 15 h. Après purification comme précédemment, on isole 0,05g (0,38 mmol) de 2,6-difluorophénol 4b (éluant : éther/éther de pétrole = 10/90) que l'on contrôle par CPG semi-capillaire avec le produit commercial 4b d'Aldrich (26,446-6). Le rendement en composé 4b est de 20 %.

### b) A partir du diol 3e

Même mode opératoire que pour 3a : effectué sur 0,69 g (3,12 mmol) de diol difluoré 3e, 10 équivalents de pyridine (≃ 2,5 cm³), 2g de tamis moléculaire 4Ä ; reflux 3h15. Après purification (comme pour 4a), on isole 0,29g de phénol 4b (éluant : éther/pentane = 8/92).
On contrôle en CPG semi-capillaire le produit obtenu avec le produit commercial Aldrich (26,446-6). Le rendement en composé 4b est de 71,5 %.
- F°C = 38-41.:
- RMN¹H (CDCl₃) :: 7,2-6,5 (m, 3H) ; 4,7 (s, 1H mobile). IR (CDCl₃) en cm⁻¹ : v _{OH} = 3570 (large) ;v _{C=C} = 1610.
- Référence :: Aldrich 26,446-6

### Préparation du 2-fluorophénol 4c

**1er essai** : A 0,95 g (4,63 mmol) d'alcool 1c on ajoute à la spatule 0,11g (0,2 eq) de DMAP, chauffe pendant 1h30 à 230°-240°C (bain extérieur), rajoute 0,11 g (0,2 eq) de DMAP et chauffe de nouveau à la même température pendant 4h30. Après refroidissement, on traite par HCl 10 % jusqu'à pH = 1, extrait à l'éther, sèche sur sulfate de magnésium, filtre et évapore. On purifie le produit brut par chromatographie éclair sur silice (éluant: éther/éther de pétrole = 9/91). On isole 0,2g de 2-fluorophénol 4c (Rdt=14%) et 0,35 g d'alcool allylique fluoré 5b (Rdt = 45 %).
**2ième essai** : A 0,5 g (2,44 mmol) d'alcool 1c on ajoute 0,30g (1 eq) de DMAP. On agite le milieu réactionnel jusqu'à dissolution ( 15 mn) puis chauffe à 220-230°C pendant 19H. On traite par de l'acide chlorhydrique à 10 % jusqu'à pH = 1, extrait à l'éther, sèche sur sulfate de magnésium, filtre et évapore ; le produit brut est purifié par chromatographie éclair sur silice (éluant : acétate d'éthyle / éther de pétrole = 4,5/95,5).
   On obtient 0,08g (0,73 mmol) de 2 fluorophénol 4c (Rdt = 30 %) et 0,06g (0,37 mmol) d'alcool allylique 5b, Rdt = 15 %).
d = 1,256 Eb°C = 171-172°/741 mmHg F°C = 16,1
- RMN¹H (CDCl₃) :: 7,30-6,55 (m, 4H) ; 5,27 (s, 1H mobile).
- IR en cm⁻¹ (film) :: v _{OH} = 3690-3000 v _{C=C} = 1595-1620.
- Référence :: Aldrich F1, 280-4.
Alcool allylique 5b
- RMN¹H (CDCl₃) :: 6,00-5,85 (t,1H) ; 4,25-4,00 (t,1H) ; 3,80-3,35 (s, 1H mobile) ; 2,45-1,65 (m, 4H).
- RMN¹³C (CDCl₃) :: 128,42 (s, C₂); 127,70 (s,C₃); 121,22 (t, C₆, J=244 Hz); 70,83 (t,C₁, J = 30,9 Hz); 25,04 (t,C₅, J = 24 Hz); 22,72 (t,C₄, J=4Hz);

### Trichloro-2,2,6 fluoro-6 cyclohexanol (TRANS) 1f

A 12,12 g (65,50 mmol) d'époxyde 2b (contenant 7 % de son régioisomère 2b') en solution dans 80 cm³ de dichlorométhane anhydre, on introduit à 4°C, sous atmosphère d'argon, 36 cm³ de trichlorure de bore en solution molaire dans le dichlorométhane (36 mmol). On laisse alors remonter le milieu réactionnel à température ambiante et maintient l'agitation pendant 1h. On hydrolyse avec 75 cm³ d'eau distillée et extrait (4 x 50 cm³) au dichlorométhane, regroupe et sèche sur MgSO₄ les phases organiques, évapore. Le produit brut obtenu contient les alcools diastéréoisomères 1f et 1b en proportion 1f/1b = 93/7 (détermination par CPG "macrobore"). On purifie par chromatographie éclair sur silice (éluant:éther/éther de pétrole = 4/96). On isole 10,73 g (rdt = 74 %) d'alcool 1f pur et 1,65 g (rdt = 11 %) du mélange des alcools 1f et 1b en proportion 1f/1b = 90/10. Le rendement global de la réaction est de 85,5%.

### Caractéristiques du diastéréoisomère 1f :

PM : 221,486
RMN¹H : 4,15(t, 1H, J=7,6 Hz); 3,10(d, 1H mobile, J= 7,6 Hz); 2,86-1,67 (m,6H).
RMN F₁₉ : -98 (S, 1F)
IR en cm⁻¹ (film): ^{v}OH = 3510
SM (m/e) EI (30 eV) : 226-224-222-220 (M⁺, 0,4-2,6,-8,2-8,5); 208-206-204-202- (0,4-3,1-10,2-11,3) ; 188-186-184 (5,1-25,3-39,1) : 156 (22,8) ; 155 (23,2); 154 (35,8) ; 153 (33,5); 149 (19,1); 124 (18,5); 122(50,1) ; 120(81,2); 112 (24,5); 110 (34,6); 109 (17,1); 106 (15,5); 101 (33,4); 93 (49,3); 91 (100,0) ; 85 (41,5); 75 (85,68); 65 (30,6); 59 (17,7).

| Microanalyse : C₇H₉Cl₃FO | | | | |
|---|---|---|---|---|
| Tr. % | C | 32,62 | H | 3,69 |
| Calc. % | | 32,53 | | 3,64 |

### Dichloro-1,3 fluoro-3 oxa-7 bicyclo [4.1.0] heptane 2e

A 1,6 g (7,22 mmol) d'alcool 1f pur en suspension dans 22 cm³ d'eau distillée, on ajoute en trois fois à 15-20°C 0,85 g (21,3 mmol) de soude en perle. On maintient le milieu réactionnel 1h15 à température ambiante, ajoute ensuite 10 cm³ d'éther, poursuit l'agitation pendant 5 mn puis décante et extrait la phase aqueuse à l'éther (5x25 cm³). On regroupe les phases éthérées, sèche sur sulfate de magnésium, filtre et évapore le solvant. On obtient 1,28 g d'un liquide, l'époxyde 2e ; le rendement brut est de 96 % (pureté en CPG "macrobore" > 98 %). PM : 185,026
RMN¹H : 3,69 (d,1H,J_{HF} = 4,3 Hz); 2,80-1,10 (m, 6H).
IR en cm⁻¹ (film) : 1410-1130-1110-1090-1080-920-870-845-820-790.
SM (m/e) 30 ev EI : 151-149 (M+ -Cl, 16,9-54,8) ; 124 (8,3) ; 122 (15,0); 121 (8,8); 120 (8,3); 112 (29,9) ; 110 (44,8) ; 109 (7,6); 108 (13,3); 107 (12,6); 106 (39,0); 103 (7,9); 101 (24,3) ; 93 (33,7); 91 (32,8); 86 (8,7); 85 (63,4); 75 (100,0); 65 (26,6); 59 (29,0); 55 (20,9).

| Microanalyse : C₆H₇Cl₂FO | | | | |
|---|---|---|---|---|
| Tr. % | C | 38,76 | H | 3,76 |
| Calc. % | | 38,95 | | 3,81. |

### Dichloro-2,6 difluoro-2,6 cyclohexanol 1g.

A 1,89 g (7,03 mmol) d'époxyde 2e en solution dans 20 cm³ de dichloro-1,2 éthane, on ajoute 0,47 cm³ (0,55 éq.) de BF₃, Et₂O. On porte à reflux sous atmosphère d'argon le milieu réactionnel pendant 1h45, puis hydrolyse à chaud avec 30 cm³ d'eau distillée (on maintient l'agitation pendant 10 mn). On extrait au dichlorométhane (4x15 cm³) puis sèche sur MgSO₄, filtre et évapore. On obtient 1,43 g d'alcool 1g dont la pureté en CPG "macrobore" est supérieure à 97 %. Le rendement en alcool 1g est de 98%.
- PM :: 205,033
- RMN¹H :: 4,07 (dt, 1H, J_{HF} = 8,3 Hz, J=8,5Hz); 3,07 (s, 1H mobile, J=8,5 Hz); 2,90-1,40 (m,6H).
- IR en cm⁻¹ (film) :: v_{OH} = 3530,3420
- SM (m/e) EI 30 ev :: 208-206-204 (M+, 0,66-3,35-5,17); 190-188-186 (0,43-2,81-4,54); 170-168 (7,1-20,9); 139 (10,9); 138 (19,7); 137 (28,4); 133 (14,5); 129 (10,2); 122 (10,3); 121 (15,3); 120 (25,2); 113 (16,1); 106-104 (18,2-52,6); 96 (10,6); 94 (16,1); 93 (29,7); 91 (31,5); 88 (17,4); 85(64,7); 75 (100,0); 65 (20,9); 59 (34,1).

### Dichloro-2,6 difluoro-2,6 cyclohexanediol-1,1 3e

A 1,41 g (7,48 mmol) d'alcool 1g en solution dans 25 cm³ de dichloroéthane, on ajoute à la spatule 4,03 g (18,69 mmol) de PCC (en maintenant une vive agitation du milieu réactionnel). On porte à reflux pendant 6 h, laisse refroidir, reprend à l'éther et filtre sur florisil. La solution obtenue est évaporée et le produit obtenu incorporé sur silice. On purifie par chromatographie éclair sur silice (éluant éther/éther de pétrole = 10/90 puis 20/80) : on obtient une première fraction contenant 0,24 g d'alcool 1g de départ (17 %) et une seconde contenant 1,01 g du diol-1,1 3e attendu. Le rendement en produit 1g est de 66,5 %.
PM : 221,03
RMN¹H : 3,96 (s,2H mobile S, J= 8,5 Hz); 2,70-1,60 (m, 6H).
IR en cm-¹ (film pur) : v_{OH} = 3520 (très large).
RMN¹³C (CDCl₃) 20 MHz : 110,58 (d,C₂ et C₆; J = 251,4 Hz); 91,31 (t, C₁, J = 22,1 Hz); 35,61 (d,C₃ et C₅, J= 22 Hz); 17,51 (d, C₄, J = 4,9 Hz).

| Microanalyse : C₆H₈Cl₂ F₂O₂ | | | | |
|---|---|---|---|---|
| tr. % | C | 32,15 | H | 3,79 |
| Calc. % | | 32,60 | | 3,65 |

### Chloro-3-difluoro-1,3-oxa-7-bicyclo [4,1,0] heptane 2f

Même ride opératoire que pour 2e effectué avec 0,18g (0,88 mmol) d'alcool 1g pur, 3 cm d'eau distillée et 0,12 g (3 mmol) de soude, temps de réaction 1h15; extraction à l'éther (3x12 cm). Après traitement, on obtient 0,13 g d'époxyde 2f (pureté CPG "macrobore" > 98 %). Le rendement en époxyde 2f est de 81 %.
- PM :: 168,572
- RMN¹HH :: 3,73 (t, 1H, J = 2,4 Hz); 2,50-1,40 (m, 6H).
- IR en cm⁻¹ (film) :: 1450-1420-1360-1340-1240-1180-1170-1100-1080-970-930-850.

### Dichloro-2,6-difluoro-2,6-cyclohexanol 1h

A 0,11g (0,65 mmol) d'époxyde 2f en solution dans 3 cm³ de dichlorométhane, on additionne à 4°C sous atmosphère d'argon 0,36 cm³ (0,55 éq.) de BCl₃ en solution molaire dans le dichlorométhane. On laisse le milieu réactionnel remonter à température ambiante (1h) puis ajoute 3 cm³ d'eau distillée et extrait au dichlorométhane (4x10 cm³). On sèche sur MgSO₄, filtre et évapore. Le produit brut est purifié par chromatographie éclair sur silice (éluant éther/éther de pétrole = 5/95). On obtient 0,08g d'alcool 1h soit un rendement de 60 % (pureté CPG "macrobore" > 95 %).
- PM :: 205,033
- RMN¹H :: 4,08 (q, 1H, J_{HF} = 5 Hz); 2,82 (s,1H mobile, J = 5Hz); 2,70-1,35 (m, 6H).

### Bromo-2-dichloro-2,6-fluoro-6-cyclohexanol 1i

A 0,98 (5,3 mmol) d'époxyde 2e en solution dans 6,5 cm³ de dichlorométhane, on introduit à 4°C sous atmosphère d'argon 2,91 cm³ (2,91 mmol) de BBr₃ en solution molaire dans le dichlorométhane. On laisse alors remonter le milieu réactionnel à température ambiante et poursuit l'agitation (1h30). On ajoute alors 5 cm³ d'eau distillée et extrait au dichlorométhane (4x10 cm³). On sèche sur MgSO₄, filtre et évapore. Le produit brut est purifié par chromatographie éclair sur silice (éluant éther/éther de pétrole = 5/95): on obtient 1,03g d'alcool 1i (pureté CPG "macrobore" >97 %), le rendement en produit 1i est de 73 %.
- PM :: 265,943
- RMN¹H :: 4,05 (t, 1H, J = 6,3 Hz); 3,17 (s, 1H, mobile, J= 6,3 Hz); 3,00-1,50 (m, 6H).
- IR en cm⁻¹ (film) :: v _{OH} = 3495.

### Bromo-1-Chloro-3-fluoro-3-oxa-7 bicyclo [4,1,0] heptane 2g

Même mode opératoire que pour 2e effectué avec 0,20 g (0,75 mmol) d'alcool 1i, 3cm³ d'eau et 0,12 g (3mmol) de soude. Durée de réaction : 1h15; extraction à l'éther (4x7 cm³). Après traitement, on obtient 0,15g d'époxyde 2g (pureté CPG "macrobore" > 97,5 %), soit un rendement en composé 2g de 80 %.
- PM :: 229,476
- RMN¹H :: 3,75 (d,1H,J = 4,2 Hz); 2,90-1,40 (m, 6H).
- IR en cm⁻¹ (film) :: 1410-1335-1240-1070 (large)-910-860-840-790-770.

| Microanalyse : C₆H₇BrClFO | | | | |
|---|---|---|---|---|
| Tr. | %C | 32,07 | H | 3,47 |
| Calc. | % | 31,40 | | 3,07. |

### Méthyl-1 tétrachloro-2,2,6,6 cyclohexanol 1j

Dans un bicol de 50 cm³ séché à l'étuve, muni d'un thermomètre et sous atmosphère d'argon, on a pesé 3,78 g (16 mmol) de tétrachloro-2,2,6,6 cyclohexanone. On introduit 29 cm³ de THF anhydre et refroidit le milieu réactionnnel à -30°C. On additionne alors 14 cm³ (16,8 mmol) d'iodure de méthyl magnésium environ 1,2 M dans l'éther en contrôlant la température.
On laisse sous agitation magnétique 15 mn puis remonte à 0°C en 45 mn. On ajoute alors à 0°C 10 cm³ d'une solution aqueuse d'acide chlorhydrique 1N, extrait à l'éther, sèche sur MgSO₄, filtre et évapore. On obtient 3,97g d'une huile qui cristallise à froid, soit un rendement brut en composé 1j de 98 %. L'alcool 1j peut être purifié par chromatographie éclair sur silice (éluant éther/éther de pétrole = 90/10).
F = 82°C
PM : 251,969 RMN¹H : 2,95-1,50 (m, 6H); 2,80 (s, 1H mobile) ; 1,92 (s,3H).
RMN¹³ C (acétone D₆): 95,7 (C₂ et C₆) ; 80,6 (C₃ et C₅) ; 20,7 (C₄) ; 18,8 (C₇).
IR en cm-¹ : v_{OH} = 3580
SM (m/e)EI : 258-256-254-252-250 (M+, 0,19-0,45-1,65-3,23-2,68); 203-201-199-197 (0,6-2,0-5,7-5,9); 175-173-171-169 (0,6-2,2-5,6-5,7); 130-128-126 (2,85-15,55-23,9); 107-105 (33,05-100,00); 92 (7,67); 77 (6,8); 65 (4,2); 45 (10,0); 43 (66,5); 41 (5,4).

| Microanalyse : C₇H₁₀Cl₄O | | | | |
|---|---|---|---|---|
| Tr. % | C | 33,37 | H | 4,00 |
| Calc. % | | 33,49 | | 4,08 |

### Trichloro-1,3,3 méthyl-2 oxa-7 bicyclo [4.1.0] heptane 2h

Même mode opératoire que pour 2e effectué avec 3,97 g (15,75 mmol) d'alcool brut 1j, 20 cm³ d'eau distillée et 2,4 g (60 mmol) de soude ; durée de réaction 1h15 ; extraction à l'éther (5x40 cm³); on obtient 3,29 g d'époxyde 2h soit un rendement de 95,5 % à partir de la cétone (pureté en CPG "macrobore" > 99 %)
Eb = 47°C/0,35 mmHg
PM : 215,506
RMN¹H (CCl₄) : 2,80-1,35 (m, 6H); 1,84 (s, 3H)
IR en cm⁻¹ (film) : 1445-1340-1080-980-945-860-760-715-665
RMN¹³ C (C₆D₆) : 9,10 (C₃) ; 85,8 (C₁); 68,3 (C₂); 40,2 (C₄); 32,8 (C₆) ; 18,6 (C₅) ; 14,9 (C₇).

| Microanalyse : C₇H₉Cl₃O | | | | |
|---|---|---|---|---|
| Tr. % | C | 39,64 | H | 4,52 |
| Calc.% | | 39,01 | | 4,21. |

### Vinyl-1 tétrachloro-2,2,6,6 cyclohexanol 1k

A 18,4 ml (16,9 mmol) de bromure de vinyl magnésium 0,9M dans le THF, on additionne sous atmosphère d'argon et au pousse seringue en 5 mn à température ambiante 2g (8,28 mmol) de tétrachloro-2,2,6,6 cyclohexanone en solution dans 8 cm₃ de THF anhydre (la température du milieu réactionnel s'élève alors jusqu'à environ 50°C). On poursuit l'agitation pendant 3h à température ambiante. On introduit 16 cm³ d'eau puis rajoute 15 cm³ d'une solution aqueuse d'acide chlorhydrique 1N. On extrait à l'éther (3x50cm³), sèche sur MgSO₄, filtre et évapore. Le produit brut est purifié par chromatographie éclair sur silice (éluant : éther/éther de pétrole = 5/95). On obtient 3 fractions : 0,05 g d'époxyde 2i pur, 0,36 g d'un mélange époxyde 2i/alcool 1k = 39/61 (détermination par CPG "macrobore")et 1,18g d'alcool 1k pur. Le rendement global en époxyde 2i est donc de 11 %, celui en alcool 1k de 63 %.

### Caractéristiques de l'époxyde 2i : voir paragraphe suivant.

### Caractéristiques de l'alcool 1k :

F = 79°C
PM : 263,98
RMN¹H : 6,95-6,55 (dd, 1H) ; 5,85-5,55 (m, 2H);2,90 (s, 1H mobile); 2,95-1,30 (m, 6H).

| Microanalyse : C₈H₁₀Cl₄O | | | | |
|---|---|---|---|---|
| Tr. % | C | 36,80 | H | 3,69 |
| Calc. % | | 36,40 | | 3,82. |

### Trichloro-1,3,3 vinyl-2 oxa-7 bicyclo [4.1.0] heptane 2i

Même mode opératoire que pour 2e effectué avec 1,14g (4,32mmol) d'alcool 1k pur, 12 cm³ d'eau distillée et 0,5g (12,5 mmol) de soude; durée de réaction 1h15; extraction à l'éther (4x25 cm³); le produit brut est utilisé tel quel (rdt brut = 90,5 %) ou purifié par chromatographie éclair sur sllice (éluant éther/éther de pétrole = 3/97): On obtient 0,88g d'époxyde 2i soit un rendement de 90 % (pureté en CPG "macrobore" >98,6 %)
- PM :: 227,519
- RMN¹H :: 6,75-6,30 (m, 1H); 5,80-5,45(m, 2H); 3,00-1,25 (m, 6H).
- RMN¹³C :: 126,1 (C₇); 123,1 (C₈); 88,35 (C₃); 85,5 (C₁); 70,0 (C₂); 39,75 (C₄); 32,4 (C₆); 18,3 (C₅).
- IR en cm⁻¹ (film) :: 1080-940-875-785-735.

### Allyl-1 tétrachloro-2,2,6,6 cyclohexanol 1l

On a pesé dans un bicol de 25 cm³ séché à l'étuve, muni d'un thermomètre et sous atmosphère d'argon, 0,95g (4,03 mmol) de 2,2,6,6-tétrachlorocyclohexanone. On ajoute 6 cm³ de THF anhydre et refroidit le milieu réactionnel à -30°C. On additionne alors 13 cm³ (4,8 mmol) de bromure d'allyl magnésium (0,37 M) en contrôlant la température à -30°C, laisse réagir 30 mn et hydrolyse entre -25°C et -10°C par une solution de chlorure d'ammonium à 5%. On extrait 5 fois à l'éther, sèche, filtre et évapore.
On obtient 1,12g d'une huile cristallisant à froid ; l'alcool 1l; le rendement brut est de 100 %.
- F = 50°C:
- PM :: 278,010
- RMN¹H :: 6,20-5,65 (m, 1H) ; 5,30-4,95 (m, 2H) ; 3,20-3,00 (d, 2H, J = 6,5 Hz et s, 1H mobile) ; 2,90-1,35 (m, 6H).
- RMN¹³C :: 133,3 (C₉); 122,1 (C₈); 95,4 (C₂ et C₆); 79,5 (C₁); 42,2 (C₃ et C₅); 36,8 (C₇) ; 20,5 (C₄).
- IR en cm⁻¹ (film) :: v_{OH} = 3520; v_{C} = C = 1640-915.

### Allyl-2 trichloro-1,3,3 oxa-7 bicyclo [4.1.0] heptane 2j

Même mode opératoire que pour 2e effectué avec 1,12 g (4,03 mmol) d'alcool 1l brut, 5,5 cm³ d'eau distillée et 0,6 g (12,5 mmol) de soude; durée de réaction 1h15; extraction à l'éther ; le produit brut est utilisé tel quel (rdt brut = 90,5 %) ou purifié par chromatographie éclair sur silice (éluant éther/éther de pétrole = 3/97) : On obtient 0,89 g d'époxyde 2j soit un rendement de 92 % (pureté en CPG "macrobore" > 98,6 %).
PM : 241,545
RMN¹H : 6,25-4,70 (m, 3H) ; 3,10 (d,2H, J = 7Hz); 2,85-1,10 (m,6H).
RMN¹³C : 132,5 (C₈) ; 118,0 (C₉); 90,6 (C₃) ; 85,0 (C₁); 68,4 (C₂); 40,0 (C₇); 32,5 (C₄ et C₆); 18,4 (C₅).
IR en cm⁻¹ (film) : 3080-1640-1430-1080-920-875-780-730.

| Microanalyse : C₇H₉Cl₃O | | | | |
|---|---|---|---|---|
| Tr. % | C | 45,01 | H | 4,32 |
| Calc. % | | 44,75 | | 4,59. |

### Trichloro-2,2,6 fluoro-6 méthyl-1 cyclohexanol 1m

Dans un flacon de 20 cm³ en polyéthylène, muni d'une agitation magnétique, d'un septum et d'une sortie vers la hotte aspirante, on introduit 4 cm³ de complexe HF/pyridine (Aldrich 18,422-5). On additionne en 20 mn 1,92g (8,91 mmol) d'époxyde 2h à -10°C, laisse réagir à cette température pendant 1h10 puis laisse revenir le milieu réactionnel à température ambiante (10 mn). On hydrolyse à -5°C par 12cm³ d'eau distillée (5 mn), extrait au dichlorométhane (4x20 cm³), sèche sur MgSO₄, filtre et évapore. Le produit brut est purifié par chromatographie éclair sur silice (éluant éther / éther de pétrole = 5 / 95) : On obtient 1,79g d'un mélange de diastéréoisomères des alcools 1m/1m' = 95 / 5 (détermination par CPG "macrobore"). Le rendement global de la réaction est de 85,5 %.

### Caractéristiques de l'isomère majoritaire 1m :

F = 95°C (mélange 1m / 1m')
PM : 235,515
RMN¹H : 2,90 (s, 1H mobile); 2,85-1,60 (m, 6H); 1,75 (s, 3H).
RMN¹³C (100 MHz) : 115,2 (d, C₂, J = 245 Hz); 95,39 (s, C₆); 80,01 (d, C₁, J = 19 Hz); 42,31 (s, C₅) ; 36,64 (d,C₃, J = 21 Hz); 19,79 (s, C₇); 19,39 (s,C₄).
IR en cm⁻¹ (film) : v_{OH} = 3560.

| Microanalyse : C₇H₁₀Cl₃FO | | | | |
|---|---|---|---|---|
| Tr. % | C | 35,80 | H | 4,15 |
| Calc. % | | 35,70 | | 4,28. |

### Dichloro-3,3 fluoro-1 méthyl-2 oxa-7 bicyclo [4.1.0] heptane 2k

Même mode opératoire que pour 2e effectué avec 1,68g (7,13 mmol) d'alcool 1m , (contenant 5 % de régioisomère 1m'), 12 cm³ d'eau distillée et 1g (25 mmol) de soude ; durée de réaction 1h15; extraction à l'éther (4x25 cm³); on obtient 1,35g d'un mélange brut (pureté CPG "macrobore" globale > 99,7 %) d'époxydes 2k, 2k', 2k'' en proportions respectives 2k/2k'/2k'' = 89/4,5/6,5 soit un rendement brut de 95 %.

### Caractéristiques de l'isomère majoritaire 2k

PM : 199,054
RMN¹H : 2,50-1,60 (m, 6H); 1,75 (s, 3H).
IR en cm⁻¹ (film): 1260-1195-1160-1105 à 1075-930-885-880-770-730.

| Microanalyse : C₇H₉Cl₂FO | | | | |
|---|---|---|---|---|
| Tr. | %C | 42,38 | H | 4,49 |
| Calc. | | 42,24 | | 4,56 |

### Dichloro-2,2 fluoro-6,6 méthyl-1 cyclohexanol 1n

Même mode opératoire que pour 1m effectué avec 1g (5,02 mmol) du mélange d'époxydes 2k / 2k' /2k'' = 89/4,5/6,5 additionné en 16 mn à 0°C, laisse réagir à cette température pendant 20 mn puis laisse revenir le milieu réactionnel à température ambiante (1h15). Le produit brut est purifié par chromatographie éclair sur silice (éluant: éther/éther de pétrole = 5 /95) : On obtient 0,94 g d'un mélange des alcools 1n/1n'/1n'' = 89/4,5/6,5 (détermination par CPG "macrobore"). Le rendement global de la réaction est de 85,5 %.

### Caractéristiques de l'isomère majoritaire 1n :

F= 58,59° C (mélange 1n/1n'/1n'')
PM : 219,060
RMN¹H : 2,50 (s, 1H mobile); 3,05-1,45 (m, 6H); 1,65 (s, large, 3H).
IR en cm⁻¹ : v_{OH} = 3590.

| Microanalyse : C₇H₁₀Cl₂F₂O | | | | |
|---|---|---|---|---|
| Tr. % | C | 39,14 | H | 4,78 |
| Calc. % | | 38,38 | | 4,60. |

### Chloro-1 difluoro-3,3 méthyl-2 oxa-7 bicyclo [4.1.0] heptane 2l

Même mode opératoire que pour 2e effectué avec 1,53 g (6,98 mmol) du mélange d'alcools 1n/1n'/1n'' = 89 /4,5/6,5, 10 cm³ d'eau distillée et 0,8g (20 mmol) de soude ; durée de réaction 1h15 ; extraction à l'éther (4x15 cm³); on obtient 1,19 g d'un mélange brut (pureté CPG "macrobore" globale > 99 %) d'époxydes 2l, 2l', 2l'' non séparables en CPG "macrobore" et donc en proportion supposée 2l/2l'/2l'' = 89/4,5/6,5 soit un rendement brut (pur) de 93 %.
PM : 182,599
RMN¹H : 2,79-1,42 (m, 6H); 1,65 (s, 3H).
IR en cm⁻¹ (film) : 1360-1330-1265-1200-1165-1100-1040-990-960-915-890-860.

| Microanalyse : C₇H₉ClF₂O | | | | |
|---|---|---|---|---|
| Tr. % | C | 45,74 | H | 5,17 |
| Calc. % | | 46,05 | | 4,97. |

### Chloro-3 phénol 4d

1,01g (5,01 mmol) d'époxyde 2a en solution dans 3 cm³ de DMF anhydre sont portés à reflux pendant 3h. On acidifie (pH = 1) avec HCl 3N, extrait (4x20 cm³) à l'éther, sèche sur MgSO₄, filtre et évapore. Le produit brut obtenu est purifié par chromatographie éclair sur silice (éluant : éther/éther de pétrole = 10/90). On obtient 0,55g de phénol 4d le rendement en composé 4d est de 83,5 % (pureté CPG "macrobore" > 95%).
- F = 33-35°C:
- Eb = 214°C:
- PM :: 128,559
- RMN¹H :: 7,5-6,5 (m,4H) : 6,0 (s, large, 1H mobile).
- IR en cm⁻¹ :: v_{OH} = 3550 (large) : ^{v}C = C = 1590.
- Produit Aldrich :: ref. C_{6,280-8.}

### Mise en évidence de la dichloro-2,3 cyclohex-2-ène-1-one 8 :

Lors de la purification sur silice, on isole une fraction contenant environ 8mg d'un produit (pureté de 71 % en CPG "macrobore") dont l'analyse (RMM¹H 80 MHz, IR et une CPG / masse ) permet de penser qu'il s'agit de la dichloro-2,3 cyclohex-2-ène-1-one 8.
- PM :: 165,020
- RMN¹H :: 2,95-2,45 (m, 4H); 2,35-1,85 (q, 2H).
- IR en cm⁻¹ :: v_{C = O} = 1690 ; v_{C = C} = 1590.
- SM (70 ev) m/e :: 168-166-164 (M⁺ ; 5,2-31,6-51,6); 140-138-136 (10,6-62,7-100,0); 129 (13,6); 110 (15,5); 108 (22,2); 103-101 (5,9-19,1); 75 (13,4); 73 (35,5); 65 (26,9); 55 (39,6).

### Chloro-3 méthyl-2 phénol 4e

2 g (9,28 mmol) d'époxyde 2h en solution dans 5 cm³ de DMF anhydre sont portés à reflux pendant 3h. On acidifie (pH = 1) avec HCl 3N, extrait (4x25 cm³) à l'éther, sèche sur MgSO₄, filtre et évapore. Le produit brut obtenu est purifié par chromatographie éclair sur silice (éluant : éther/éther de pétrole = 5/95). On obtient 1,18g de phénol 4e (pureté CPG "macrobore" > 96 %). Le rendement en composé 4e est de 89%.
F = 84°C.
PM : 142,586
RMN¹H (CCl₄) : 7,10-6,75 (d, 2H); 6,75-6,40 (m, 1H); 4,90 (s, 1H mobile); 2,20 (s, 3H).
RMN¹³C (CCl₄) : 154,2 (C₁) : 135,2 (C₃); 126,7 (C₅); 122,7 (C₂); 121,6 (C₄); 113,3 (C₆) ; 12,3 (C₇).
IR en cm⁻¹ (CCl₄): v_{OH} = 3600; v_{C} = _{C} = 1585.

| Microanalyse : C₇H₇ClO | | | | |
|---|---|---|---|---|
| Tr. % | C | 58,37 | H | 5,06 |
| Calc. % | | 58,96 | | 4,94. |

### Chloro-3 vinyl-2 phénol 4f

0,84g (3,69 mmol) d'époxyde 2i en solution dans 4 cm³ de DMF anhydre sont portés à reflux pendant 3h30. On acidifie (pH = 1) avec HCl 3N, extrait (4x20 cm³) à l'éther, sèche sur MgSO₄, filtre et évapore. Le produit brut obtenu est purifié par chromatographie éclair sur silice (éluant : éther / éther de pétrole = 5 /95). On obtient 0,43g de phénol 4f (pureté CPG "macrobore" > 99 %). Le rendement en composé 4f est de 75,5 %.
F = 48°C.
PM : 154,597
RMN¹H : 7,25-6,55 (m, 4H); 5,85-5,55 (m, 3H dont 1H mobile).
IR en cm⁻¹ : v_{OH} = 3260 : v_{C=C} = 1630, 1595, 1585.

| Microanalyse : C₈H₇ClO | | | | |
|---|---|---|---|---|
| Tr. % | C | 62,15 | H | 4,56 |
| Calc. % | | 62,30 | | 4,48. |

### Allyl-2 chloro-3 phénol 4g

2g (8,28 mmol) d'époxyde 2j en solution dans 5 cm³ de DMF anhydre sont portés à reflux pendant 5h. On acidifie (pH < 1) avec HCl 3N, extrait à l'éther, concentre et ajoute à la phase éthérée une solution de soude 3N (en maintenant le milieu agité pendant 5 à 10 mn), élimine la phase éthérée et lave une fois à l'éther. On acidifie la phase aqueuse par une solution avec HCl 3N (pH = 1) et extrait (4x30 cm³) à l'éther, sèche sur MgSO₄, filtre et évapore. On obtient 1,24g de phénol 4g, liquide dont la pureté en CPG "macrobore" est supérieure à 97 %. Le rendement en phénol 4g est de 89%.
PM : 168,624
RMN¹H : 7,05-6,50 (m, 3H); 6,20-5,65 (m,1H); 5,60-4,85 (s,1H mobile) 3,55 (d, 2H, J = 6,1 Hz).
RMN¹³C : 154, 85 (C₁) ; 134,9 (C₃); 134,5 (C₈); 127,6 (C₅); 123,95 (C₂); 121,8 (C₄); 115,85 (C₉); 114,1 (C₆); 31,2 (C₇).
IR en cm⁻¹ (film) : v_{OH} = 3500; v_{C} = C = 3080-1640-1580-910.
SM (m/e) EI : 170-168 (M+, 32,2-100,0); 155-153 (17,8-59,0); 143-141 (6,6-22,9); 133 (43,2); 132 (9,4); 127-125 (10,8-34,1); 115 (24,9); 105 (37,7); 103 (18,7); 79 (11,7); 78 (11,9); 77 (43,8); 63 (10,5); 51 (33,3); 49 (10,4).

| Microanalyse : C₉H₉ClO | | | | |
|---|---|---|---|---|
| Tr. % | C | 63,80 | H | 5,26 |
| Calc.% | | 64,11 | | 5,38. |

### Fluoro-3 méthyl-2 phénol 4h

0,65g (3,69 mmol) d'époxyde 2l (mélange 2l, 2l', 2l'' précédent) en solution dans 3 cm³ de DMF anhydre sont portés à reflux pendant 3h : on détecte alors par CPG macrobore 91 % de cétone de transposition 6b (vérification par couplage masse/CPG). On ajoute alors 0,86g (2 éq.) de DMAP et porte de nouveau à reflux pendant 3h. On acidifie (pH = 1) avec HCl 3N, extrait à l'ether, concentre et ajoute à la phase éthérée une solution de soude 3N (en maintenant le milieu agité pendant 5 à 10 mn), élimine la phase éthérée et lave une fois à l'éther. On acidifie la phase aqueuse par une solution avec HCl 3N (pH = 1) et extrait (4x15cm³) à l'éther, sèche sur MgSO₄, filtre et évapore. Le produit brut obtenu est purifié par chromatographie éclair sur silice (éluant : éther/ éther de pétrole = 5 / 95). On obtient 0,10g de phénol 4h (pureté CPG "macrobore" >97 %). Le rendement en composé 4h est de 22 %.
- PM :: 126,132
- RMN¹H :: 7,15-6,30 (m, 3H); 5,15 (s,1H mobile); 2,14 (d, 3H, J = 2Hz).
- IR en cm⁻¹ :: v_{OH} = 3450 (large); v_{C} = C = 1620 (faible), 1595.
- SM (m/e) :: 126 (M⁺, 100); 125 (87); 109 (10,5); 108 (28); 107 (14,5); 97 (30,5); 95(10); 77 (16,5); 49 (21).

### Fluoro-3 phénol 4i

0,90g (4,86 mmol) d'époxyde 2e en solution dans 3 cm³ de DMF anhydre sont portés à reflux pendant 3h. On acidifie (pH = 1) avec HCl 3N, extrait (4x20 cm³) à l'éther, sèche sur MgSO₄, filtre et évapore. Le produit brut obtenu est purifié par chromatographie éclair sur silice (éluant : éther / éther de pétrole = 10 / 90). On obtient 0,53g d'un mélange des phénols 4i et 4d en proportion 4i/4d = 76 / 24. Le rendement brut global est de 85 %. Les phénols 4i et 4d sont identifiés par rapport aux produits commerciaux de chez Aldrich (par CPG "macrobore").
- Eb = 178°C:
- PM :: 112,105
- RMN¹H :: 7,40-6,40 (m, 4H); 5,70 (s, large, 1H mobile).
- IR en cm⁻¹ (film):: v_{OH} = 3350; v_{C = C} = 1600
- Référence :: Aldrich F1, 300-2.

### Mise en évidence de l'allyl-2 trichloro-2,3,3 cyclohexanone 6a

1,60g (6,62 mmol) d'époxyde 2j et 2 cm³ (25 mmol) de pyridine sont portés à reflux pendant 24h. Après refroidissement, on acidifie jusqu'à pH=1 par HCl 3N, extrait (3x30 cm³) à l'éther, sèche sur MgSO₄, filtre et évapore. Le produit brut est purifié par chromatographie éclair sur silice (éluant : éther/éther de pétrole = 1 / 99). On isole (dans l'ordre d'élution) de l'époxyde 2j de départ, 0,40g de cétone de transposition 6a (rdt = 40 %), des traces d'allyl-2 dichloro-2,3 cyclohexène-3 one 7 (mise en évidence par CPG / masse) et du phénol 4f.

### Caractéristiques de l'allyl-2 trichloro-2,3,3 cyclohexanone 6a :

F = 42-43°C.
PM : 241,546
RMN¹H : 6,1-5,5 (m, 1H); 5,3-4,9 (m, 2H); 3,8-1,7 (m,6H); 2,97 (d,2H,J = 7,3 Hz).
RMN¹³C (100 MHz) : 198,93 (C₁); 132,38 (C₉); 118,98 (C₈); 95,30 (C₃); 76,89 (C₂); 41,40 (C₄); 37,99 (C₇ ou C₆); 34,78 (C₆ ou C₇); 21,07 (C₅).
IR en cm⁻¹ (film) : v_{C = O} = 1740; v_{C = C} = 3080-1645-920.
SM (m/e) EI : 244-242-240 (M+, 0,68-1,36-1,31); 209-207-205 (5,8-26,7-45,0); 171-169 (33,5-100,0); 166-164-162 (8,1-40,3-67,1); 149 (14,2); 147 (17,0); 141 (15,1); 135 (24,1); 133 (14,6); 129-127 (28,1-81,3); 115 (16,7); 113 (39,7); 107 (10,8); 105 (41,05); 99 (10,0); 91 (52,1); 79 (52,6); 78 (16,0); 77 (75,0); 75 (15,1); 65 (23,1); 63 (14,0); 55 (82,9); 53 (17,4); 52 (14,5); 51 (41,7); 49 (11,0).

| Microanalyse : C₉H₁₁Cl₃O | | | | |
|---|---|---|---|---|
| Tr. % | C | 44,47 | H | 4,89 |
| Calc. | | 44,75 | | 4,59. |

### Caractéristiques de l'allyl-2-dichloro-2,3-cyclohex-3-ène-1-one 7 :

- PM :: 205,085
- RMN¹H :: 6,1-5,4(m,1H); 5,2-4,8(m, 2H); 4,3 (t,1H,J = 5,2Hz); 3,2 (d,1H, J=6,5 Hz); 3,1-1,6(m,6H).
- SM (m/e):: 208-206-204 (M⁺, 1,3-7,5-10,8); 171-169 (32,0-100,0); 153-151 (8,7-17,0); 144-142 (13,3-39,7); 133 (36,0); 127 (16,1); 114 (12,2); 107 (34,1); 105(23,1), 79 (81,8), 77 (37,9).

### Allyl-2 chloro-3 phénol 4g à partir de la cétone 6a

0,13g (0,54 mmol) de cétone 6a en solution dans 1 cm³ de DMF anhydre sont portés à reflux pendant 10h. Après refroidissement, on acidifie par HCl 3N, extrait à l'éther. On contrôle alors par CPG "macrobore" que le produit majoritaire (rendement CPG > 90 %) est bien identique au phénol 4g préparé précedemment.

### Trichloro-2,2,6-fluoro-6-cyclohexanone 3a'

0,45g (1,81 mmol) d'hydrate 3a (dosage RMN¹⁹F : hydrate 3a/cétone 3a'= 90,4/9,6) en solution dans 10 cm³ de benzène anhydre sont introduits dans un Dean-Stark muni d'une agitation magnétique et sous atmosphère d'argon. On porte à reflux pendant 1 nuit (en fin de réaction on constate par mesure IR effectuée dans le benzène l'apparition d'une bande carbonyle vers 1765 cm⁻¹) et après refroidissement du milieu réactionnel, évapore le benzène. On obtient 0,28g de cétone 3a' (dosage RMN¹⁹F : hydrate 3a/cétone 3a' = 98,5/1,5).
Le rendement de la réaction est de 70 % en composé 3a'.
- F°C = 34:
- PM :: 219,47
- RMN¹H 200 MHz :: 2,95-2,75 (m,1H): 2,75-2,35 (m,3H); 2,3-1,9 (m,2H).
- RMN¹⁹F (CDCl₃) 190 MHz :: -104,00 (t,0,94F, (J = 10,1 Hz); -105,7 (t, 0,06F, J=10,1, Hz), (D, C₁, J = 22,5 Hz);
- RMN¹³C (CDCl₃) 50 MHz :: 185,02.
104,97 (d, C₆, J = 257,8 Hz); 84,13 (C₂); 46,46 (C₃); 41,10 (d, C₅, J = 21,2 Hz); 18,32 (d, C₄, J = 7 Hz).
- IR en cm⁻¹ (C₆H₆):: v_{C=O} : 1765.
- SM (m/e):: 224-222-220-218 (M+, 0,29-2,04-6,28-6,65);
194-192-190 (1,20-3,94-3,95); 156-154-152 (2-10,13- 14,83); 146 (6,4); 144 (5,7); 121-119 (32,0-100,0); 111 (9,2); 109 (14,9); 93 (22,9); 83 (16,7); 77 (13,5); 75 (43,7)-58 (13,5); 56 (24,8); 53 (10,1). Balayage jusqu'à m/e = 50.

## Revendications

1. Composé caractérisé par le fait qu'il répond à la formule générale (II) suivante : 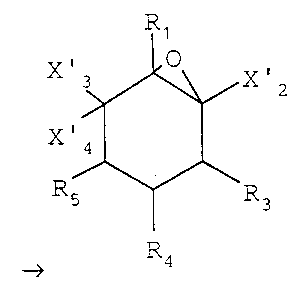
• dans laquelle X'₂, X'₃ et X'₄ (qui correspondent un à un à X₂, X₃ et X₄ ou X₁, X₂ et X₃ de la formule I) semblables ou différents représentent un halogène ou un pseudo-halogène, de préférence un halogène avantageusement le chlore et le fluor avec la condition que lorsque R₁ est hydroxyle, cyano, amido, imido, éthoxyle, benzyloxyle, cyclohexyle et tertiobutyle, tous les halogènes ne peuvent être simultanément du chlore et R₄, R₃ et R₅ simultanément égal à H ;
• dans laquelle R1 représente un hydrogène, une chaîne hydrocarbonée telle que par exemple une chaîne alcoyle, une chaîne aromatique ou un groupement alcoxyle, carboxyle ou acyloxyle, voire hydroxyle ;
• dans laquelle R4 représente un hydrogène, un atome de fluor, une chaîne hydrocarbonée telle que par exemple une chaîne alcoyle, une chaîne aromatique ou un groupement carboxyle, carboxamide ou bien un radical relié au cycle hexanique par un chalcogène ou par élément de la colonne V de préférence de la première période, tel qu'un groupement amido, alcoxyle ou acyloxyle ;
• dans laquelle les radicaux R3 et R5 différents ou de préférence semblables, représentent un atome de fluor, ou de préférence d'hydrogène, ou également des chaînes hydrocarbonées telles que définies ci-dessus pour R4.

2. Composé selon la revendication 1, caractérisé par le fait que le nombre de carbones par substituants n'excède pas 15, avantageusement 10.

3. Procédé de préparation des composés selon l'une des revendications 1 et 2, caractérisé par le fait qu'il comporte une étape d'action d'une base sur un alcool de formule générale (I') ci-après : 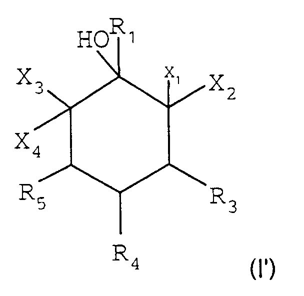
dans laquelle X₁, X₂, X₃ et X₄ semblables ou différents représentent un halogène ou un pseudo-halogène, avantageusement un halogène de préférence le chlore et le fluor ;
dans laquelle R₁ représente un hydrogène, une chaîne hydrocarbonée telle que par exemple une chaîne alcoyle, une chaîne aromatique ou un groupement alcoxyle, carboxyle ou acyloxyle, hydroxyle ;
dans laquelle R₄ représente un hydrogène, un atome de fluor, une chaîne hydrocarbonée telle que par exemple une chaîne alcoyle, une chaîne aromatique ou un groupement carboxyle, carboxamide ou bien un radical relié au cycle hexanique par un chalcogène ou par élément de la colonne V de préférence de la première période, tel qu'un groupement amido, alcoxyle ou acyloxyle ;
dans laquelle les radicaux R3 et R5 différents ou de préférence semblables, représentent un atome de fluor, ou de préférence d'hydrogène, ou également des chaînes hydrocarbonées telles que définies ci-dessus pour R4.

4. Procédé selon la revendication 3 , caractérisé par le fait que la dite étape est réalisé dans une phase polaire, de préférence aqueuse.

5. Procédé selon la revendication 4, caractérisé par le fait que ladite phase polaire est susceptible de dissoudre à la fois le substrat alcoolique (I') et la base ; avantageusement la solubilité du substrat alcoolique étant au moins égale à 10-3 M plus préférentiellement à 10-2 M et celle de la base à 10-3 N plus préférentiellement à 10-2 N ;

6. Procédé selon l'une des revendications 3 à 5, caractérisé par le fait que la dite étape est réalisée à une température comprise entre le point de fusion commençante du mélange réactionnel et 100°C de préférence entre O°C et 50°C, en général entre 0°C et l'ambiante (environ 20°C).

7. Procédé selon l'une des revendications 3 à 6, caractérisé par le fait que ladite base est une base forte dont le pKa associé est au moins égal à 10 de préférence à 12, plus préférentiellement très forte.

8. Procédé de synthèse d'un composé aromatique présentant une fonction phénol halogénée ou pseudo halogénée en méta, caractérisé par le fait qu'il comporte l'étape d'aromatisation d'un composé répondant à la formule générale (II) suivante : 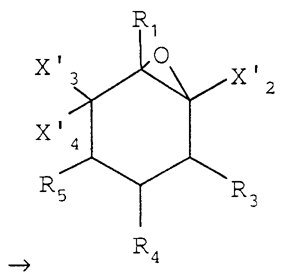
• dans laquelle X'₂, X'₃ et X'₄ (qui correspondent un à un à X₂, X₃ et X₄ ou X₁, X₂ et X₃ de la formule I) semblables ou différents représentent un halogène ou un pseudo-halogène, de préférence un halogène avantageusement le chlore et le fluor avec la condition que lorsque R₁ est cyano et tertiobutyle, tous les halogènes ne peuvent être simultanément du chlore et R₄, R₃ et R5 simultanément égal à H ;
• dans laquelle R1 représente un hydrogène, une chaîne hydrocarbonée telle que par exemple une chaîne alcoyle, une chaîne aromatique ou un groupement alcoxyle, carboxyle ou acyloxyle, voire hydroxyle ;
• dans laquelle R4 représente un hydrogène, un atome de fluor, une chaîne hydrocarbonée telle que par exemple une chaîne alcoyle, une chaîne aromatique ou un groupement carboxyle, carboxamide ou bien un radical relié au cycle hexanique par un chalcogène ou par élément de la colonne V de préférence de la première période, tel qu'un groupement amido, alcoxyle ou acyloxyle ;
• dans laquelle les radicaux R₃ et R₅ différents ou de préférence semblables, représentent un atome de fluor, ou de préférence d'hydrogène, ou également des chaînes hydrocarbonées telles que définies ci-dessus pour R₄ et par le fait que ladite l'étape d'aromatisation est réalisée par réalisée par action à chaud de systèmes de solvant(s) à caractère basique.

9. Procédé selon la revendication 8, caractérisé par le fait que lesdits solvant(s) à caractère basique sont mis en oeuvre en présence de tamis moléculaire.

10. Procédé selon la revendication 8 et 9, caractérisé par le fait que lesdits solvant(s) à caractère basique sont choisis parmi les amines, sels d'amines, phosphines et amides.

11. Procédé selon la revendication 8 à 10, caractérisé par le fait que lesdits systèmes de solvant(s) sont associés à des sels tels que des halogénures d'alcalins tels que LiCl ou de sels d'acides faibles tels que les carbonates.

12. Procédé selon la revendication 8 à 11, caractérisé par le fait que lesdits solvant(s) à caractère basique sont choisis parmi les amides.

13. Procédé selon la revendication 8 à 12, caractérisé par le fait que lesdits solvant(s) à caractère basique sont choisis parmi ceux à noyaux aromatiques basiques.

14. dérivés cyclohexaniques présentant la formule (I) ci-après : 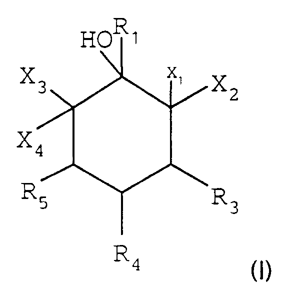
• Dans laquelle X₁, X₂, X₃ et X₄ semblables ou différents représentent un halogène ou un pseudo-halogène, avantageusement un halogène de préférence le chlore et le fluor ;
• dans laquelle R₁ représente un hydrogène, une chaîne hydrocarbonée telle que par exemple une chaîne alcoyle, une chaîne aromatique ou un groupement alcoxyle, carbonyle, carboxyle ou acyloxyle, hydroxyle ;
• dans laquelle R₄ représente un hydrogène, un atome de fluor, une chaîne hydrocarbonée ;
• dans laquelle les radicaux R3 et R5 différents ou de préférence semblables, représentent un atome de fluor, ou de préférence d'hydrogène, ou également des chaînes hydrocarbonées ;
avec la condition supplémentaire que lorsque R1 est hydroxyle, tous les halogènes ne peuvent être simultanément du chlore ou du brome et lorsque R1 est hydrogène, cyano, amido, tous les halogènes ne peuvent être simultanément du chlore.

15. Composé selon la revendication 14, caractérisé par le fait que le nombre de carbones par substituants n'excède 15 avantageusement 10.

16. Procédé d'obtention des produits selon les revendications 14 et 15 caractérisé par le fait qu'il est choisi selon l'une des 2 méthodes suivantes :
a) Traitement d'un époxyde de formule générale II par ouverture au moyen d'un ion halogénure ou pseudo-halogénure.
b) Par réduction d'un composé de formule I avec R1 = OH.

17. Procédé selon la revendication 15, caractérisé par le fait que l'on utilise la méthode a) et par le fait que l'on utilise comme source d'halogénure ou de pseudo-halogénure, un acide de Bronstedt ou de Lewis.

18. Procédé selon la revendication 17 caractérisé par le fait que ledit acide de Lewis est choisi parmi les trihalogénures des éléments de la colonne III B de la classification périodique des éléments (définie par la Société Chimique de France, dans le supplément au Bull. Soc. Chim. Fr. N°1 Janvier 1966), et notamment ceux susceptibles d'exister sous la forme de divers étherates.

19. Procédé selon la revendication 17, caractérisé par le fait que les acides de Bronstedt préférés sont choisis parmi ceux dont l'ion halogénure ou pseudohalogénure constitue la base associée, avantageusement en présence d'une amine faible, de préférence les amines réputées tertiaires et qui contiennent comme source de base un atome d'azote inséré dans un cycle aromatique.

20. Procédé selon la revendication 15 caractérisé par le fait que l'on utilise la méthode b) et par le fait que l'on utilise comme réactif un hydrure double d'un métal alcalin et d'un élément de la colonne III B de la classification périodique des éléments tels que publié dans le Bull. Soc. Chim. Fr. N°1, 1966 ou bien un halogénure d'alcoyle secondaire magnésium.

21. Procédé selon la revendication 20 caractérisé par le fait que l'on utilise comme réducteur le borohydrure de sodium en présence d'un mélange éther-alcool.

22. Procédé selon la revendication 21 caractérisé par le fait que la réaction est menée à une température comprise entre 0 et 50°C, de préférence entre 15 et 30°C.

23. Procédé selon la revendication 21 et 22 caractérisé par le fait que le rapport préféré entre l'éther et l'alcool, exprimé en volume, est avantageusement compris entre 10 et 1, de préférence aux alentours de 10.

24. Procédé de synthèse d'un composé aromatique halogéné ou pseudo halogénés, caractérisé par le fait qu'il comporte l'étape d'aromatisation d'un composé cétonique répondant à la formule générale (I') suivante : 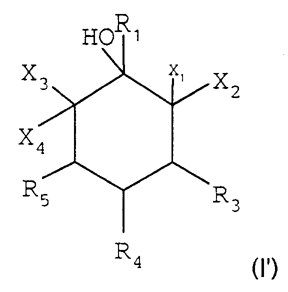
• dans laquelle X₁, X₂, X₃ et X₄ semblables ou différents représentent un halogène ou un pseudo-halogène, de préférence un halogène avantageusement le chlore et le fluor avec la condition que lorsque R₁ est cyano et tertiobutyle, tous les halogènes ne peuvent être simultanément du chlore et R₄, R₃ et R5 simultanément égal à H ;
• dans laquelle R1 représente un hydroxyle ;
• dans laquelle R4 représente un hydrogène, un atome de fluor, une chaîne hydrocarbonée telle que par exemple une chaîne alcoyle, une chaîne aromatique ou un groupement carbonyle, carboxyle, carboxamide ou bien un radical relié au cycle hexanique par un chalcogène ou par élément de la colonne V de préférence de la première période, tel qu'un groupement amido, alcoxyle ou acyloxyle ;
• dans laquelle les radicaux R3 et R5 différents ou de préférence semblables, représentent un atome de fluor, ou de préférence d'hydrogène, ou également des chaînes hydrocarbonées telles que définies ci-dessus pour R4 ;
ou d'un composé équivalents au composés cétonique ci dessus ; et par le fait que ladite étape d'aromatisation est réalisée par action à chaud de systèmes de solvant(s) à caractère basique

25. Procédé selon la revendication 24, caractérisé par le fait que ladite l'étape d'aromatisation est réalisée en présence d'agent deshydratant, et notamment de tamis moléculaire.

26. Procédé selon la revendication 24 et 24, caractérisé par le fait que lesdits solvant(s) à caractère basique sont choisis parmi les amines, sels d'amines, phosphines et amides.

27. Procédé selon la revendication 24 à 25, caractérisé par le fait que lesdits systèmes de solvant(s) sont associés à des sels tels que des halogénures d'alcalins tels que LiCl ou de sels d'acides faibles tels que les carbonates.

28. Procédé selon les revendications 24 à 26, caractérisé par le fait que lesdits solvant(s) à caractère basique sont choisis parmi les amides.

29. Procédé selon les revendications 24 à 27, caractérisé par le fait que lesdits solvant(s) à caractère basique sont choisis parmi ceux à noyaux aromatiques basiques.

30. Procédé permettant d'accéder à des dérivés benzèniques halogénés caractérisé par le fait qu'il comporte une succession de formation et d'ouverture d'époxyde selon respectivement les revendications 2 à 7 et 17 à 19

31. Procédé selon la revendication 30, caractérisé par le fait qu'il comporte une étape d'aromatisation en elle même connue ou selon les revendications 8 à 13 ou 24 à 29.

## Claims

1. Compound, characterized in that it corresponds to the following general formula (II): 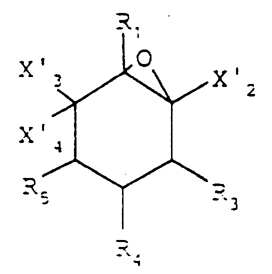
• in which X'₂, X'₃ and X'₄ (which correspond one to one to X₂, X₃ and X₄ or X₁, X₂ and X₃ in the formula I), which are identical or different, represent a halogen or a pseudohalogen, preferably a halogen, advantageously chlorine and fluorine, with the condition that, when R₁ is hydroxyl, cyano, amido, imido, ethoxy, benzyloxy, cyclohexyl and tert-butyl, the halogens cannot all be chlorine simultaneously and R₄, R₃ and R₅ cannot be simultaneously equal to H;
• in which R₁ represents a hydrogen, a hydrocarbon chain such as, for example, an alkyl chain, an aromatic chain or an alkoxy, carboxyl or acyloxy, indeed hydroxyl, group;
• in which R₄ represents a hydrogen, a fluorine atom, a hydrocarbon chain such as, for example, an alkyl chain, an aromatic chain or a carboxyl or carboxamide group or alternatively a radical connected to the hexane ring via a chalcogen or via an element of group V, preferably of the first period, such as an amido, alkoxy or acyloxy group;
• in which the R₃ and R₅ radicals, which are different or preferably identical, represent a fluorine atom or, preferably, a hydrogen atom or also hydrocarbon chains, as defined above for R₄.

2. Compound according to claim 1, characterized in that the number of carbons per substituent does not exceed 15 and advantageously does not exceed 10.

3. Process for the preparation of compounds according to one of claims 1 and 2, characterized in that it comprises a stage of reaction of a base with an alcohol of general formula (I') below: 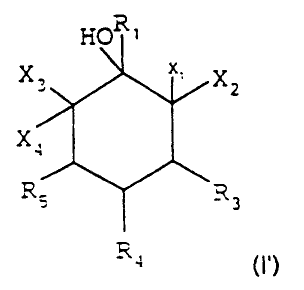
in which X₁, X₂, X₃ and X₄, which are identical or different, represent a halogen or a pseudohalogen, advantageously a halogen, preferably chlorine and fluorine;
in which R₁ represents a hydrogen, a hydrocarbon chain such as for example, an alkyl chain, an aromatic chain or an alkoxy, carboxyl or acyloxy, or hydroxyl group;
in which R₄ represents a hydrogen, a fluorine atom, a hydrocarbon chain such as, for example, an alkyl chain, an aromatic chain or a carboxyl or carboxamide group or alternatively a radical connected to the hexane ring via a chalcogen or via an element of group V, preferably of the first period, such as an amido, alkoxy or acyloxy group;
in which the R₃ and R₅ radicals, which are different or preferably identical, represent a fluorine atom or, preferably, a hydrogen atom or also hydrocarbon chains, as defined above for R₄.

4. Process according to claim 3, characterized in that the said stage is carried out in a polar phase, preferably an aqueous phase.

5. Process according to claim 4, characterized in that the said polar phase is capable of dissolving both the alcoholic substrate (I') and the base; the solubility of the alcoholic substrate advantageously being at least equal to 10⁻³ M, more preferentially to 10⁻² M, and that of the base advantageously being at least equal to 10⁻³ N, more preferentially to 10⁻² N.

6. Process according to one of claims 3 to 5, characterized in that the said stage is carried out at a temperature between the temperature at which the reaction mixture begins to melt and 100°C, preferably between 0°C and 50°C, generally between 0°C and room temperature (approximately 20°C).

7. Process according to one of claims 3 to 6, characterized in that the said base is a strong base, the associated pKₐ of which is at least equal to 10, preferably to 12, and more preferentially a very strong base.

8. Process for the synthesis of an aromatic compound exhibiting a phenol functional group which is halogenated or pseudohalogenated in the meta position, characterized in that it comprises the stage of aromatization of a compound corresponding to the following general formula (II): 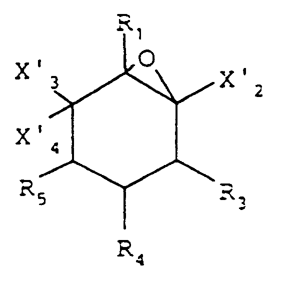
• in which X'₂, X'₃ and X'₄ (which correspond one to one to X₂, X₃ and X₄ or X₁, X₂ and X₃ in the formula I), which are identical or different, represent a halogen or a pseudohalogen, preferably a halogen, advantageously chlorine and fluorine, with the condition that, when R₁ is cyano and tert-butyl, the halogens cannot all be chlorine simultaneously and R₄, R₃ and R₅ cannot be simultaneously equal to H;
• in which R₁ represents a hydrogen, a hydrocarbon chain such as, for example, an alkyl chain, an aromatic chain or an alkoxy, carboxyl or acyloxy, indeed hydroxyl, group;
• in which R₄ represents a hydrogen, a fluorine atom, a hydrocarbon chain such as, for example, an alkyl chain, an aromatic chain or a carboxyl or carboxamide group or alternatively a radical connected to the hexane ring via a chalcogen or via an element of group V, preferably of the first period, such as an amido, alkoxy or acyloxy group;
• in which the R₃ and R₅ radicals, which are different or preferably identical, represent a fluorine atom or, preferably, a hydrogen atom or also hydrocarbon chains, as defined above for R₄, and in that the said aromatization stage is carried out by the effect while hot of systems containing solvent(s) with a basic nature.

9. Process according to claim 8, characterized in that the said solvent(s) with a basic nature are used in the presence of a molecular sieve.

10. Process according to claims 8 and 9, characterized in that the said solvent(s) with a basic nature are chosen from amines, amine salts, phosphines and amides.

11. Process according to claims 8 to 10, characterized in that the said systems containing solvent(s) are used in combination with salts, such as alkali metal halides, such as LiCl, or salts of weak acids, such as carbonates.

12. Process according to claims 8 to 11, characterized in that the said solvent(s) with a basic nature are chosen from amides.

13. Process according to claims 8 to 12, characterized in that the said solvent(s) with a basic nature are chosen from those with basic aromatic nuclei.

14. Cyclohexane derivatives exhibiting the formula (I) below: 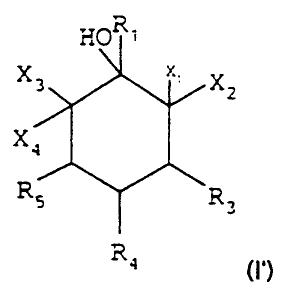
• in which X₁, X₂, X₃ and X₄, which are identical or different, represent a halogen or a pseudohalogen, advantageously a halogen, preferably chlorine and fluorine;
• in which R₁ represents a hydrogen, a hydrocarbon chain such as, for example, an alkyl chain, an aromatic chain or an alkoxy, carbonyl, carboxyl or acyloxy, or hydroxyl group;
• in which R₄ represents a hydrogen, a fluorine atom or a hydrocarbon chain;
• in which the R₃ and R₅ radicals, which are different or preferably identical, represent a fluorine atom or, preferably, a hydrogen atom or also hydrocarbon chains; with the additional condition that, when R₁ is hydroxyl, the halogens cannot all be chlorine or bromine simultaneously and, when R₁ is hydrogen, cyano or amido, the halogens cannot all be chlorine simultaneously.

15. Compound according to claim 14, characterized in that the number of carbons per substituent does not exceed 15 and advantageously does not exceed 10.

16. Process for obtaining products according to claims 14 and 15, characterized in that it is chosen according to one of the 2 following methods:
a) Treatment of an epoxide of general formula II with opening by means of a halide or pseudohalide ion.
b) By reduction of a compound of formula I with R₁ = OH.

17. Process according to claim 16, characterized in that the method a) is used and in that a Bronstedt or Lewis acid is used as source of halide or of pseudohalide.

18. Process according to claim 17, characterized in that the said Lewis acid is chosen from trihalides of elements of group IIIb of the Periodic Classification of the Elements (defined by the Société Chimique de France, in the supplement to the Bull. Soc. Chim. Fr., No.1, January 1966) and in particular those capable of existing in the form of various etherates.

19. Process according to claim 17, characterized in that the preferred Bronstedt acids are chosen from those in which the halide or pseudohalide ion constitutes the associated base, advantageously in the presence of a weak amine, preferably amines considered to be tertiary and which contain, as source of base, a nitrogen atom inserted into an aromatic ring.

20. Process according to claim 16, characterized in that the method b) is used and in that use is made, as reagent, of a double hydride of an alkali metal and of an element of group IIIb of the Periodic Classification of the Elements, as published in Bull. Soc. Chim. Fr., No.1, 1966, or alternatively of a secondary alkylmagnesium halide.

21. Process according to claim 20, characterized in that use is made, as reducing agent, of sodium borohydride in the presence of an ether/alcohol mixture.

22. Process according to claim 21, characterized in that the reaction is carried out at a temperature of between 0 and 50°C, preferably between 15 and 30°C.

23. Process according to claims 21 and 22, characterized in that the preferred ratio of the ether to the alcohol, expressed by volume, is advantageously between 10 and 1, preferably in the region of 10.

24. Process for the synthesis of a halogenated or pseudohalogenated aromatic compound, characterized in that it comprises the stage of aromatization of a ketone compound corresponding to the following general formula (I'): 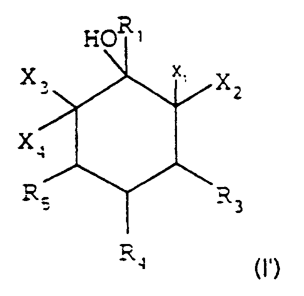
• in which X₁, X₂, X₃ and X₄, which are identical or different, represent a halogen or a pseudohalogen, preferably a halogen, advantageously chlorine and fluorine, with the condition that, when R₁ is cyano and tert-butyl, the halogens cannot all be chlorine simultaneously and R₄, R₃ and R₅ cannot be simultaneously equal to H;
• in which R₁ represents a hydroxyl;
• in which R₄ represents a hydrogen, a fluorine atom, a hydrocarbon chain such as, for example, an alkyl chain, an aromatic chain or a carbonyl, carboxyl or carboxamide group or alternatively a radical connected to the hexane ring via a chalcogen or via an element of group V, preferably of the first period, such as an amido, alkoxy or acyloxy group;
• in which the R₃ and R₅ radicals, which are different or preferably identical, represent a fluorine atom or, preferably, a hydrogen atom or also hydrocarbon chains, as defined above for R₄;
or of a compound equivalent to the above ketone compounds; and in that the said aromatization stage is carried out by the effect while hot of systems containing solvent(s) with a basic nature.

25. Process according to claim 24, characterized in that the said aromatization stage is carried out in the presence of a dehydrating agent and in particular of a molecular sieve.

26. Process according to claims 24 and 25, characterized in that the said solvent(s) with a basic nature are chosen from amines, amine salts, phosphines and amides.

27. Process according to claims 24 to 26, characterized in that the said systems containing solvent(s) are used in combination with salts, such as alkali metal halides, such as LiCl, or salts of weak acids, such as carbonates.

28. Process according to claims 24 to 27, characterized in that the said solvent(s) with a basic nature are chosen from amides.

29. Process according to claims 24 to 28, characterized in that the said solvent(s) with a basic nature are chosen from those with basic aromatic nuclei.

30. Process allowing access to halogenated benzene derivatives, characterized in that it comprises an epoxide formation and opening sequence according to claims 3 to 7 and 16 to 19 respectively.

31. Process according to claim 30, characterized in that it comprises an aromatization stage known in itself or according to claims 8 to 13 or 24 to 29.

## Patentansprüche

1. Verbindung, dadurch gekennzeichnet, daß sie der folgenden allgemeinen Formel (II) entspricht: 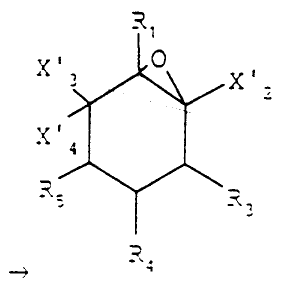
. worin X'₂, X'₃ und X'₄ (welche nacheinander X₂, X₃ und X₄ oder X₁, X₂ und X₃ der Formel I entsprechen), welche gleich oder verschieden voneinander sind, ein Halogenatom oder ein Pseudohalogenatom, vorzugsweise ein Halogenatom, vorteilhaft Chlor und Fluor bedeuten, mit der Bedingung, daß wenn R₁ eine Hydroxyl-, Cyan-, Amid-, Imid-, Ethoxy-, Benzyloxy-, Cyclohexyl- und tert.-Butylgruppe ist, alle Halogenatome nicht gleichzeitig Chlor und R₄, R₃ und R₅ nicht gleichzeitig Wasserstoff sein dürfen;
. worin R₁ ein Wasserstoffatom, eine Kohlenwasserstoffkette, wie beispielsweise eine Alkylgruppe eine aromatische Kette oder eine Alkoxy-, Carboxy- oder Acyloxy- sogar Hydroxylgruppe bedeutet;
. worin R₄ ein Wasserstoffatom, ein Fluoratom, eine Kohlenwasserstoffkette, wie beispielsweise eine Alkylkette, eine aromatische Kette oder eine Carboxyl-, Carboxamidgruppe oder einen Rest darstellt, der durch ein Chalkogen oder durch ein Element der Gruppe V, vorzugsweise der ersten Periode mit dem Cyclohexanring verbunden ist, wie eine Amid-, Alkoxy- oder Acyloxygruppe;
. worin die Reste R₃ und R₅, welche gleich oder vorzugsweise verschieden sind, ein Fluoratom oder vorzugsweise ein Wasserstoffatom oder auch Kohlenwasserstoffketten, wie sie vorstehend für R₄ definiert sind, bedeuten.

2. Verbindung nach Anspruch 1, dadurch gekennzeichnet, daß die Anzahl von Kohlenstoffen pro Substituenten 15, vorteilhafterweise 10, nicht übersteigt.

3. Verfahren zur Herstellung von Verbindungen gemäß einem der Ansprüche 1 und 2, dadurch gekennzeichnet, daß es einen Schritt der Einwirkung einer Base auf einen Alkohol der nachfolgenden allgemeinen Formel (I') umfaßt: 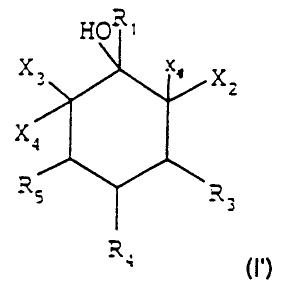
worin X₁, X₂, X₃ und X₄, welche gleich oder verschieden voneinander sind, ein Halogenatom oder ein Pseudohalogenatom, vorteilhafterweise ein Halogenatom, vorzugsweise Chlor und Fluor bedeuten;
worin R₁ ein Wasserstoffatom, eine Kohlenwasserstoffkette, wie beispielsweise eine Alkylkette, eine aromatische Kette oder eine Alkoxy-, Carboxyl- oder Acyloxy-, Hydroxylgruppe bedeutet;
worin R₄ ein Wasserstoffatom, ein Fluoratom, eine Kohlenwasserstoffkette, wie beispielsweise eine Alkylkette, eine aromatische Kette oder eine Carboxyl-, Carboxamidgruppe oder auch einen Rest bedeutet, der durch ein Chalkogen oder durch ein Element der Gruppe V, vorzugsweise der ersten Periode, mit dem Cyclohexanring verbunden ist, wie eine Amid-, Alkoxy- oder Acyloxygruppe;
worin die Reste R₃ und R₅, welche verschieden oder vorzugsweise gleich sind, ein Fluoratom oder vorzugsweise Wasserstoffatom oder auch Kohlenwasserstoffketten, wie sie oben für R₄ definiert sind, bedeuten.

4. Verfahren nach Anspruch 3, dadurch gekennzeichnet, daß der Schritt in einer polaren Phase, welche vorzugsweise wäßrig ist, durchgeführt wird.

5. Verfahren nach Anspruch 4, dadurch gekennzeichnet, daß die polare Phase fähig ist, gleichzeitig das alkoholische Ausgangsmaterial (I') und die Base zu lösen, wobei in vorteilhafter Weise die Löslichkeit des alkoholischen Ausgangsstoffes wenigstens 10⁻³ M, bevorzugter 10⁻² M und die der Base 10⁻³ N bevorzugter 10⁻² N ist.

6. Verfahren nach einem der Ansprüche 3 bis 5, dadurch gekennzeichnet, daß der Schritt bei einer Temperatur durchgeführt wird, welcher zwischen dem Anfangsschmelzpunkt der Reaktionsmischung und 100°C, vorzugsweise zwischen 0°C und 50°C, und im allgemeinen zwischen 0°C und Raumtemperatur (etwa 20°C) enthalten ist.

7. Verfahren nach einem der Ansprüche 3 bis 6, dadurch gekennzeichnet, daß die Base eine starke, bevorzugter sehr starke Base ist, deren verbundener pKa-Wert wenigstens gleich 10, vorzugsweise 12 ist.

8. Verfahren zur Herstellung einer aromatischen Verbindung, die in meta-Position eine Halogenphenol- oder Pseudohalogenphenolfunktion aufweist, dadurch gekennzeichnet, daß sie den Schritt zur Aromatisierung einer Verbindung umfaßt, welche der folgenden allgemeinen Formel (II) entspricht: 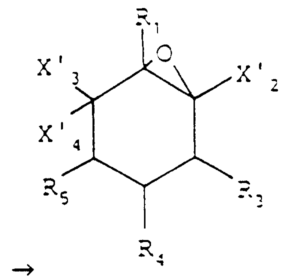
. worin X'₂, X'₃ und X'₄ (welche nacheinander X₂, X₃ und X₄ oder X₁, X₂ und X₃ der Formel I entsprechen), welche gleich oder verschieden voneinander sind, ein Halogenatom oder ein Pseudohalogenatom, vorzugsweise ein Halogenatom, vorteilhaft Chlor und Fluor bedeuten, mit der Bedingung, daß wenn R₁ eine Cyan- und tert.-Butylgruppe ist, alle Halogenatome nicht gleichzeitig Chlor und R₄, R₃ und R₅ nicht gleichzeitig Wasserstoff sein dürfen;
. worin R₁ ein Wasserstoffatom, eine Kohlenwasserstoffkette, wie beispielsweise eine Alkylgruppe, eine aromatische Kette oder eine Alkoxy-, Carboxyl- oder Acyloxy- sogar Hydroxylgruppe bedeutet;
. worin R₄ ein Wasserstoffatom, ein Fluoratom, eine Kohlenwasserstoffkette, wie beispielsweise eine Alkylkette, eine aromatische Kette oder eine Carboxyl-, Carboxamidgruppe oder auch einen Rest darstellt, der durch ein Chalkogen oder durch ein Element der Gruppe V, vorzugsweise der ersten Periode, mit dem Cyclohexanring verbunden ist, wie eine Amid-, Alkoxy- oder Acyloxygruppe;
. worin die Reste R₃ und R₅, welche verschieden voneinander oder vorzugsweise gleich sind, ein Fluoratom oder vorzugsweise Wasserstoffatom oder auch Kohlenwasserstoffketten, wie sie vorstehend für R₄ definiert sind, bedeuten und dadurch, daß der Aromatisierungsschritt durch Wärmeeinwirkung von Lösungsmittelsystemen mit basischem Charakter durchgeführt wird.

9. Verfahren nach Anspruch 8, dadurch gekennzeichnet, daß das bzw. die Lösungsmittel mit basischem Charakter in Gegenwart von Molekularsieben eingesetzt werden.

10. Verfahren nach Anspruch 8 und 9, dadurch gekennzeichnet, daß das bzw. die Lösungsmittel mit basischem Charakter aus den Aminen, Aminsalzen, Phosphinen und Amiden ausgewählt werden.

11. Verfahren nach Anspruch 8 bis 10, dadurch gekennzeichnet, daß die Lösungsmittelsysteme mit Salzen vergemeinschaftet sind, wie den Alkalihalogeniden, wie LiCl oder den Salzen von schwachen Säuren, wie den Carbonaten.

12. Verfahren nach Anspruch 8 bis 11, dadurch gekennzeichnet, daß das bzw. die Lösungsmittel mit basischem Charakter aus den Amiden ausgewählt ist bzw. sind.

13. Verfahren nach Anspruch 8 bis 12, dadurch gekennzeichnet, daß das bzw. die Lösungsmittel mit basischem Charakter aus jenen mit basischem, aromatischem Kern ausgewählt sind.

14. Cyclohexanderivat mit der folgenden Formel (I): 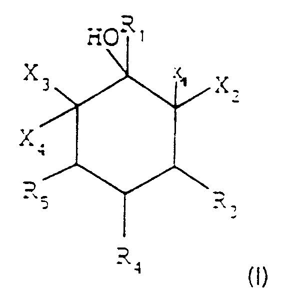
. worin X₁, X₂, X₃ und X₄, welche gleich oder verschieden voneinander sind, ein Halogenatom oder ein Pseudohalogenatom, vorteilhaft ein Halogenatom, vorzugsweise Chlor oder Fluor bedeuten;
. worin R₁ ein Wasserstoffatom, eine Kohlenwasserstoffkette, wie beispielsweise eine Alkylkette eine aromatische Kette oder eine Alkoxy-, Carbonyl-, Carboxy- oder Acyloxy-, Hydroxylgruppe bedeuten;
. worin R₄ ein Wasserstoffatom, ein Fluoratom, eine Kohlenwasserstoffkette bedeuten;
. worin die Reste R₃ und R₅, welche verschieden voneinander oder vorzugsweise gleich sind, ein Fluoratom oder vorzugsweise Wasserstoffatom oder auch Kohlenwasserstoffketten bedeuten, mit der zusätzlichen Bedingung, daß wenn R₁ eine Hydroxylgruppe ist, alle Halogenatome nicht gleichzeitig Chlor oder Brom sein dürfen und wenn R₁ ein Wasserstoffatom, eine Cyan- oder Amidgruppe ist, alle Halogenatome nicht gleichzeitig Chlor sein dürfen.

15. Verfahren nach Anspruch 14, dadurch gekennzeichnet, daß die Anzahl von Kohlenstoffen pro Substituenten 15, vorteilhaft 10, nicht übersteigt.

16. Verfahren zum Erhalten von Produkten gemäß den Ansprüchen 14 und 15, dadurch gekennzeichnet, daß es nach einem der zwei folgenden Verfahren ausgewählt ist:
a) Behandeln eines Epoxides der allgemeinen Formel II durch Öffnen mittels eines Halogen- oder Pseudohalogenions.
b) Durch Reduktion einer Verbindung der Formel I, wobei R₁ = OH ist.

17. Verfahren nach Anspruch 15, dadurch gekennzeichnet, daß man das Verfahren a) verwendet und dadurch, daß man als Halogen- oder Pseudohalogenquelle eine Broenstedt- oder Lewissäure verwendet.

18. Verfahren nach Anspruch 17, dadurch gekennzeichnet, daß die Lewissäure aus den Trihalogeniden der Elemente der Gruppe IIIB des Periodensystemes (definiert durch die Société Chimique de France, in der Beilage zu Bull. Soc. Chim. Fr. Nr. 1, Januar 1966) auswählt und insbesondere jenen, welche in Form von verschiedenen Ätheraten vorliegen können.

19. Verfahren nach Anspruch 17, dadurch gekennzeichnet, daß die bevorzugten Broenstedtsäuren aus jenen ausgewählt sind, deren Halogen- oder Pseudohalogenion die korrespondierende Base bilden, vorteilhaft in Gegenwart eines schwachen Amines, bevorzugt den tertiären Aminen, ausgewählt sind und welche als Basenquelle ein in einen aromatischen Ring eingebrachtes Stickstoffatom enthalten.

20. Verfahren nach Anspruch 15, dadurch gekennzeichnet, daß man das Verfahren b) verwendet und dadurch, daß man als Reaktiv ein Dihydrid eines Alkalimetalles und eines Elementes der Gruppe IIIB des Periodensystemes, wie es in Bull. Soc. Chim. Fr. Nr. 1, Januar 1966 veröffentlicht ist, oder auch ein Sec.-Alkylhalogenid-Magnesium verwendet.

21. Verfahren nach Anspruch 20, dadurch gekennzeichnet, daß man als Reduktionsmittel Natriumborhydrid in Gegenwart einer Äther-Alkohol-Mischung verwendet.

22. Verfahren nach Anspruch 21, dadurch gekennzeichnet, daß die Reaktion bei einer Temperatur gehalten wird, welche zwischen und 0 und 50°C, vorzugsweise zwischen 15 und 30°C, enthalten ist.

23. Verfahren nach Anspruch 21 und 22, dadurch gekennzeichnet, daß das bevorzugte Verhältnis zwischen Äther und Alkohol, ausgedrückt in Volumen vorteilhaft zwischen 10 und 1, bevorzugt etwa 10 beträgt.

24. Verfahren zur Herstellung einer halogenierten oder pseudohalogenierten aromatischen Verbindung, dadurch gekennzeichnet, daß es den Schritt zur Aromatisierung einer Ketonverbindung umfaßt, welche der folgenden allgemeinen Formel (I') entspricht: 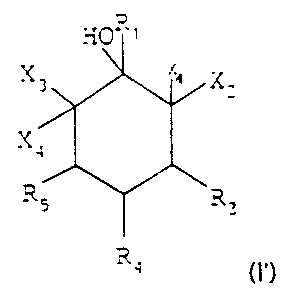
. worin X₁, X₂, X₃ und X₄, welche gleich oder verschieden voneinander sind, ein Halogenatom oder ein Pseudohalogenatom, vorzugsweise ein Halogenatom, vorteilhaft Chlor oder Fluor bedeuten, mit der Bedingung, daß wenn R₁ eine Cyan- und tert.-Butylgruppe ist, alle Halogenatome nicht gleichzeitig Chlor und R₄, R₃ und R₅ nicht gleichzeitig H sein dürfen;
. worin R₁ eine Hydroxylgruppe bedeutet;
. worin R₄ ein Wasserstoffatom, ein Fluoratom, eine Kohlenwasserstoffkette, wie beispielsweise eine Alkylkette, eine aromatische Kette oder eine Carbonyl-, Carboxyl-, Carboxamidgruppe oder auch einen Rest bedeutet, der durch eine Chalkogen oder ein Element der Gruppe V, vorzugsweise der ersten Periode, mit dem Cyclohexanring verbunden ist, wie eine Amid-, Alkoxyl- oder Acyloxylgruppe;
. worin die Reste R₃ und R₅, welche verschieden voneinander oder vorzugsweise gleich sind, ein Fluoratom oder vorzugsweise Wasserstoffatom oder auch Kohlenwasserstoffketten, wie sie oben für R₄ definiert sind, bedeuten,
oder einer Verbindung, welche zu der obigen Ketonverbindung äquivalent ist und dadurch, daß der Aromatisierungsschritt durch Wärmeeinwirkung von Lösungsmittelsystemen mit basischem Charakter durchgeführt wird.

25. Verfahren nach Anspruch 24, dadurch gekennzeichnet, daß der Aromatisierungsschritt in Gegenwart eines Dehydratisierungsmittels, und insbesondere eines Molekularsiebes durchgeführt wird.

26. Verfahren nach Anspruch 24 und 25, dadurch gekennzeichnet, daß das bzw. die Lösungsmittel mit basischem Charakter aus den Aminen, Aminsalzen, Phosphinen und Amiden ausgewählt sind.

27. Verfahren nach Anspruch 24 bis 25, dadurch gekennzeichnet, daß die Lösungsmittelsysteme mit Salzen vergemeinschaftet sind, wie den Alkalihalogeniden, wie LiCl oder den Salzen von schwachen Säuren, wie den Carbonaten.

28. Verfahren nach den Ansprüchen 24 bis 26, dadurch gekennzeichnet, daß das bzw. die Lösungsmittel mit basischem Charakter aus den Amiden ausgewählt sind.

29. Verfahren nach den Ansprüchen 24 bis 27, dadurch gekennzeichnet, daß das bzw. die Lösungsmittel mit basischem Charakter aus jenen mit einem basischen aromatischen Kern ausgewählt sind.

30. Verfahren, welches den Zugang zu Halogenbenzolderivaten erlaubt, dadurch gekennzeichnet, daß es eine Aufeinanderfolge der Bildung und Öffnung von Epoxid gemäß den Ansprüchen 2 bis 7 und 17 bis 19 umfaßt.

31. Verfahren nach Anspruch 30, dadurch gekennzeichnet, daß es einen an sich bekannten Aromatisierungsschritt umfaßt oder gemäß den Ansprüchen 8 bis 13 oder 24 bis 29 verläuft.
